# EUROPÄISCHE PATENTANMELDUNG

(11) **EP 4 529 851 A1**
(43) Veröffentlichungstag der Anmeldung: **02.04.2025**
(21) Anmeldenummer: 23200297.2
(22) Anmeldetag: 28.09.2023
(51) Int. Cl.: A61B 6/04, A61B 6/10, A61G 13/02, G05B 19/4061, A61B 6/00

(54) **BEWEGUNGSSTEUERUNG EINER MEDIZINISCHEN ANLAGE**

(71) Anmelder: Siemens Healthineers AG, 91301 Forchheim (DE)
(72) Erfinder: Karl, Harald, 90765 Fürth (DE)
(74) Vertreter: Siemens Healthineers Patent Attorneys

(57) **Zusammenfassung**

Die Erfindung betrifft ein Bewegungssteuerverfahren für eine bewegliche Komponente (BK, CA, M, PL) einer medizinischen Anlage (100), umfassend die Schritte
i) Erfassen (S01) einer dreidimensionalen Bildinformation (3DB) von der beweglichen Komponente (BK) und ihrer Umgebung (U) mittels einer Erfassungseinheit (EH)
ii) Ermitteln (S02) eines ersten Positionsparameters, (PP1) zu der beweglichen Komponente (BK) basierend auf der dreidimensionalen Bildinformation (3DB) mittels einer Recheneinheit (30),
iii) Erfassen (S03) eines zweiten Positionsparameters (PP2) zu der beweglichen Komponente (BK) mittels Recheneinheit (30),
iv) Vergleichen (S04) des ersten und des zweiten Positionsparameters (PP1, PP2) mittels Recheneinheit (30),
v) Erzeugen (S05) eines Bewegungssteuersignals (BSS) für die bewegliche Komponente (BK) basierend auf der dreidimensionalen Bildinformation (3DB), sofern die Abweichung zwischen erstem und zweitem Positionsparameter (PP1, PP2) einen vorab definierten ersten Schwellwert (S1) unterschreitet mittels Recheneinheit (30),
vi) Erzeugen (S06) eines Fehlersignals (FS), sofern die Abweichung zwischen erstem und zweitem Positionsparameter (PP1, PP2) den ersten Schwellwert (S1) überschreitet mittels Recheneinheit (30), und
vii) Ausführen (S07) des Bewegungssteuersignals (BSS) durch die bewegliche Komponente (BK) oder des Fehlersignals (FS) durch die Recheneinheit (30).

## Beschreibung

Die vorliegende Erfindung betrifft eine Bewegungssteuerung einer beweglichen Komponente einer medizinischen Anlage.

Eine medizinische Anlage im Sinne der Erfindung kann eine medizinische Anlage zur medizinischen Bildgebung und/oder zur medizinischen Behandlung oder Therapie eines Patienten sein. Darunter fallen bspw. medizinische Bildgebungsanlagen wie ein Computertomograph oder ein Magnetresonanztomograph, ein C-Bogen-Angiographiegerät, Ultraschallgeräte und/oder PET (= Positronen-Emissions-Tomographie)-Anlagen oder dergleichen. Diese Aufzählung ist jedoch nicht abschließend. Daneben kann eine medizinische Anlage als Behandlungs- oder Therapie-Anlage bspw. eine Strahlentherapie-Anlage oder ein zumindest teilweise automatisiert geführtes Interventionsinstrument ausgebildet sein. Auch diese Aufzählung ist nicht abschlie-ßend.

Eine medizinische Anlage umfasst wenigstens eine bewegliche Komponente. Diese stellt in Ausführungen eine Untereinheit bzw. einen Bestandteil der medizinischen Anlage dar, in anderen Ausführungen ist die bewegliche Komponente die gesamte medizinische Anlage. In Ausführungen ist nur eine bewegliche Komponente umfasst, in anderen Ausführungen können mehrere, bspw. zwei oder drei bewegliche Komponenten vorgesehen sein. Diese sind bevorzugt unabhängig voneinander beweglich ausgebildet. In Ausführungen kann die Beweglichkeit mehrerer beweglicher Komponenten auch eine konzertierte, also aufeinander abgestimmte Bewegbarkeit umfassen. Die Beweglichkeit der beweglichen Komponente ist im Sinne der Erfindung so zu verstehen, dass die wenigstens eine bewegliche Komponente relativ zu restlichen Bestandteilen der medizinischen Anlage und/oder zu Objekten oder Personen in der Nähe der medizinischen Anlage bewegbar ausgebildet ist, also einen Ortswechsel vollziehen kann. Anders ausgedrückt, kann eine bewegliche Komponente ihre Position in Bezug auf ein nicht näher definiertes Koordinatensystem verändern.

Eine bewegliche Komponente kann bspw. als ein Patiententisch, als ein Haltearm für eine medizinische Bildgebungskomponente, bspw. eine Röntgenstrahlenquelle, ein Haltearm für ein Interventionsinstrument, ein Angiographie-C-Bogen oder dergleichen ausgebildet sein.

Hauptzweck der Beweglichkeit ist bei medizinischen Anlagen typischerweise die Ausrichtung der beweglichen Komponente auf eine bestimmte Körperregion eines Patienten. Die Bewegung der beweglichen Komponente kann aber auch dem Transport des Patienten und/oder der medizinischen Anlage und/oder dem Transport von weiterem medizinischen Material oder Zubehör hin zu einer Zielposition dienen.

Jede Bewegung stellt ein Sicherheitsrisiko dar, da es zu Kollisionen der beweglichen Komponente mit Personen oder weiteren Objekten kommen kann, was eine Beschädigung oder Verletzung zur Folge haben kann, die es zu vermeiden gilt.

Bspw. haben Angiographiegeräte eine besonders hohe Anzahl von beweglichen Komponenten, welche mehrfach in einer Umgebung mit einer Mehrzahl von medizinischem Fachpersonal und einer Vielzahl von medizinischen Zusatzgeräten in direkter Nähe zu den bewegten Komponenten während einer Untersuchung bzw. eines medizinischen Eingriffs bewegt werden müssen. Bei Angiographiegeräten ist ein Kollisionspotential mit anderen Teilen des Systems, dem Patienten aber auch einem der Benutzer besonders groß.

Die Europäische Norm IEC60601 für medizinische elektrische Geräte umfasst die Vorgabe, Kollisionen grundsätzlich zu vermeiden. Zudem regelt sie Werte für zulässige Restkräfte im Falle einer unvermeidlichen Kollision. Ferner besteht die Anforderung, dass Mittel zur Kollisionsvermeidung erstfehlersicher ausgeführt sind.

In der Fachwelt ist es bekannt, andere Teile der medizinischen Anlage, deren Dimensionen, deren Position oder deren Bewegungstrajektorien bekannt sind, über einen Kollisionsvermeidungsalgorithmus bei der Pfadplanung einer beweglichen Komponente zu berücksichtigen. Der Kollisionsvermeidungsalgorithmus ist ausgebildet, eine geplante Bewegungsbahn auf Kollisionen mit den bekannten Geräteteilen hin zu prüfen und ggf. die Bewegungsbahn anzupassen. Derartige Kollisionsvermeidungsalgorithmen erfordern eine hohe Rechenzeit vor Ausführen der Bewegung, was zu trägen, unintuitiven Bewegungsabläufen führt.

Die Lage des Patienten auf der Patientenliege ist zwar prinzipiell bekannt, seine genaue Lage bzw. seine Abmessungen jedoch nicht. Daher muss ein Kollisionsvermeidungsalgorithmus eine Sicherheitszone um den Patienten vorsehen. Erreicht eine bewegliche Komponente diese Sicherheitszone, so wird die Bewegung gestoppt. In der Sicherheitszone wird die Bewegung nur mit reduzierter Geschwindigkeit nach vorheriger manueller Freigabe fortgesetzt.

Unbekannte, insbesondere bewegliche Objekte in der Umgebung der medizinischen Anlage, bspw. ein Benutzer oder weitere medizinische Geräte, können von einem Kollisionsvermeidungsalgorithmus bei einer vorausschauenden Bahnberechnung zur Kollisionsvermeidung bislang noch nicht berücksichtigt werden.

Deshalb verfügen die medizinischen Anlagen wenigstens an kritischen Kanten und Flächen über Kollisionssensoren, welche bei Kollision auslösen und das Gerät anhalten. Die Kollisionssensoren, bspw. Mikroschalter oder Schaltleisten, haben einen Pufferweg von der Auslösung bis zur tatsächlichen Kollision. Der in aller Regel geringe Pufferweg begrenzt die maximale Geschwindigkeit, mit welcher sich die bewegte Komponente bewegen kann. Die Kollisionssensoren sind ferner ungeeignet, eine bevorstehende Kollision zu erkennen.

Der einfache Aufbau der Kollisionssensoren ermöglicht jedoch eine erstfehlersichere Ausführung. Das gilt auch für die daran angeschlossene Auswerteelektronik, welcher typischerweise nur binäre Signale erfassen und verarbeiten muss (= Auslösen eines Bewegungsstopps).

Um die Nachteile der mittels Schalter ausgebildeten Kollisionssensoren zu umgehen, können bspw. Ultraschall-, Radar- oder kapazitive Sensoren eingesetzt werden, die ausgebildet sind, Abstände zu erfassen. Jedoch weisen diese jeweils technologiebedingte Detektionsschwächen auf, sodass sie aufgrund beobachteter Unzulässigkeiten für eine erstfehlersichere Kollisions- bzw. Annäherungserkennung ungeeignet sind.

Oben adressierte Kollisionsvermeidungsalgorithmen sind aufgrund ihrer Komplexität ebenfalls nicht erstfehlersicher ausgeführt.

Demgegenüber ist es Aufgabe der vorliegenden Erfindung, verbesserte Mittel zur Kollisionsvermeidung und Pfadplanung für eine bewegliche Komponente bereitzustellen. Insbesondere ist es eine Aufgabe der vorliegenden Erfindung, eine erstfehlersichere und damit für den Bereich der medizinischen elektrischen Geräte einsetzbare Kollisionsvorhersage berechnen zu können. Insbesondere ist es auch Aufgabe der vorliegenden Erfindung, eine zuverlässige und reproduzierbare Abstandsberechnung für eine bewegliche Komponente zu einem beliebigen Objekt in der Umgebung der beweglichen Komponente bereitzustellen, um proaktiv Kollisionen zu vermeiden.

Diese Aufgabe wird gelöst durch ein Bewegungssteuerverfahren für eine bewegliche Komponente einer medizinischen Anlage, entsprechende medizinische Anlage, entsprechendes Computerprogrammprodukt und entsprechendes computerlesbares Medium gemäß den unabhängigen Ansprüchen. Bevorzugte und/oder alternative, vorteilhafte Ausgestaltungsvarianten sind Gegenstand der abhängigen Ansprüche.

Die vorliegende Erfindung betrifft in einem ersten Aspekt ein Bewegungsteuerverfahren für eine bewegliche Komponente einer medizinischen Anlage. Das Verfahren ist ein teilweise, konkret weitgehend, in Ausführungen auch vollständig computerimplementiertes Verfahren. Das Verfahren umfasst eine Vielzahl von Schritten. Die Reihenfolge der Schritte ist durch die Reihenfolge ihrer Aufzählung nicht notwendigerweise festgelegt. In bevorzugter Ausführung werden die Schritte zeitlich korreliert und mit nur geringem zeitlichen Versatz ausgeführt. In Ausführungen können Schritte auch parallel, also zeitgleich nebeneinander ausgeführt werden.

Die vorliegende Erfindung betrifft in einem weiteren Aspekt eine medizinische Anlage, die zum Ausführen des erfindungsgemäßen Verfahrens ausgebildet ist. Die medizinische Anlage umfasst wenigstens eine bewegliche Komponente bzw. ist selbst als solche ausgebildet. Daneben umfasst die medizinische Anlage eine Erfassungseinheit und eine Recheneinheit, um damit die einzelnen Verfahrensschritte auszuführen.

Nachstehend wird die erfindungsgemäße Lösung der Aufgabe in Bezug auf das beanspruchte Verfahren als auch in Bezug auf die beanspruchte Vorrichtung beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Ein erster Schritt i) des erfindungsgemäßen Verfahrens ist auf ein Erfassen einer dreidimensionalen Bildinformation von der beweglichen Komponente und ihrer Umgebung mittels der Erfassungseinheit gerichtet.

Die dreidimensionale Bildinformation bildet wenigstens die bewegliche Komponente ab. Bevorzugt bildet die dreidimensionale Bildinformation auch wenigstens ein Objekt in der Nähe der beweglichen Komponente ab. Das Objekt kann ein Bestandteil, bspw. eine weitere bewegliche Komponente oder eine unbewegliche Komponente, der medizinischen Anlage sein. Das Objekt kann auch ein von der medizinischen Anlage losgelöstes, insbesondere ebenfalls bewegliches Objekt, insbesondere eine Person, sein. Das Objekt befindet sich in der Umgebung zu der beweglichen Komponente, insbesondere befindet es sich in direkter räumlicher Nähe zu der beweglichen Komponente.

Die dreidimensionale Bildinformation entspricht in Ausführungen einem Datensatz umfassend Daten repräsentierend die relative Lage von der beweglichen Komponente und abgebildeten Objekten in deren Umgebung, und zwar in den drei Raumdimensionen. Insbesondere ermöglicht allein die dreidimensionale Bildinformation das Ableiten eines ersten Positionsparameters zu der beweglichen Komponente. Bevorzugt ermöglicht die dreidimensionale Bildinformation auch das Ableiten eines Abstands zwischen der beweglichen Komponente und wenigstens einem der abgebildeten Objekt in deren Umgebung.

Die dreidimensionale Bildinformation umfasst in bevorzugter Ausführung eine zweidimensionale Projektionsinformation der erfassten Umgebung, wobei jedem Projektionsfeld, auch Pixel genannt, eine Tiefeninformation (entsprechend der dritten Raumdimension) zugeordnet ist.

Besonders einfach und direkt lässt sich die dreidimensionale Bildinformation mit einer Erfassungseinheit erfassen, die wenigstens eine 3D (= dreidimensional)-Kamera umfasst. Mit anderen Worten umfasst die Erfassungseinheit der medizinischen Anlage zumindest in Ausführungen eine 3D-Kamera. Besonders bevorzugt kann die Erfassungseinheit auch zwei oder mehr 3D-Kameras umfassen. Die wenigstens eine 3D-Kamera ist dabei in einem vorab definierten Abstand in der Umgebung zu der beweglichen Komponente platziert und auf diese ausgerichtet. Das Bildfeld (= field of view) der 3D-Kamera deckt jedenfalls immer die bewegliche Komponente ab. Im Falle mehrerer 3D-Kameras kann die bewegliche Komponente bzw. aus verschiedenen Blickrichtungen bzw. Perspektiven erfasst werden. Werden Objekte in der abgebildeten Umgebung aus einer Blickrichtung durch die bewegliche Komponente verdeckt, können sie mittels der weiteren 3D-Kamera erfasst werden. Alternativ kann der Einsatz mehrerer 3D-Kameras von der Größe des Bildfelds der einzelnen 3D-Kamera abhängen und dieses ergänzen. Derart wird eine vorteilhaft vollständigere Darstellung der Umgebung einschließlich beweglicher Komponente erreicht und das Kollisionsrisiko vorteilhaft reduziert.

Gegenüber anderen Sensortechnologien zur Abstandsmessung, wie bspw. Ultraschall, Radar, kapazitive Sensoren, liefert eine 3D-Kamara eine signifikant bessere Detektionsqualität, insbesondere eine verbesserte räumliche Auflösung. Sie erzeugt für ein Pixelarray (bspw.) pro Pixel einen individuellen Tiefenwert. Der Tiefenwert entsteht bereits in der Kamera und muss nicht durch aufwändige Algorithmen mit Unsicherheiten (bspw. einem Algorithmus des maschinellen Lernens) ermittelt werden. Bspw. Entsteht der Tiefenwert bei einer als Time-of-Flight (= ToF) Kamera ausgebildeten 3D-Kamera direkt im TOF-Sensor.

3D-Kameras messen mit infrarotem Licht nahe dem sichtbaren Spektrum, dessen Reflektions- und Absorbtionseigenschaften sehr gut bekannt sind. Im Gegensatz zu den anderen genannten Technologien ist daher eine Fehlerwahrscheinlichkeit für die Erkennung eines Hindernisses sehr gering.

3D-Kameras werden bereits für eine Patientenvermessung, dessen Positionierung und eine Untersuchungsplanung bzw. eine Interventionsplanung eingesetzt und haben damit bereit ein breites Anwendungsfeld in der Medizintechnik. Insofern kann die medizinische Anlage in Ausführungen vorteilhaft kostenneutral die bereits vorhandene 3D-Kamera auch zur Erfassung der dreidimensionalen Bildinformation nutzen. Auch die Teilekomplexität kann beibehalten werden. 3D-Kameras sind dadurch gekennzeichnet, dass sie bei verschiedensten Szene-Bedingungen und unterschiedlichsten Umgebungsbedingungen (bspw. Leggins aus Plastik am Bein) zuverlässig dreidimensionale Bildinformation liefern. Typischerweise stellt eine 3D-Kamera ein 2D (= zweidimensional)-RGB (= rot, gelb, blau)-Bild in einer definierten, ausreichenden Widerholrate bereit (bspw. Full-HD RGB in 30fps). Daneben stellt eine 3D-Kamera auch ein Tiefenbild, ebenfalls mit ausreichender Wiederholrate, bereit (bspw. VGA 15 fps - 30fps). Das Tiefenbild umfasst für jedes Pixel der zweidimensionalen Farbdarstellung die Information, wie weit das in dem Pixel erfasste Objekt entfernt ist, mit einer Genauigkeit von weniger als einem Prozent.

Die wenigstens eine 3D-Kamera kann in bevorzugter Ausführung als Time-of-Flight (ToF)-Kamera oder "structured light" (Stereoskopie)-Kamera ausgebildet sein.

Besonders bevorzugt ist die wenigstens eine 3D-Kamera unter bzw. an der Decke des Untersuchungsraumes mit nach unten gerichtetem Blickfeld montiert. Die 3D-Kamera kann aber auch an einer Wand mit im Wesentlichen horizontal oder schräg ausgerichtetem Bildfeld angeordnet sind. Die Positionierung der wenigstens einen 3D-Kamera richtet sich insbesondere nach der Vorgaben bzw. Bedingungen der Untersuchungsumgebung.

Die wenigstens eine 3D-Kamera benötigt eine (meist kabelgebundene) Energieversorgung und eine Datenleitung bzw. Kommunikationsschnittstelle, um die dreidimensionale Bildinformation zur Weiterverarbeitung an eine Recheneinheit zu übertragen. Die Datenleitung kann kabellos oder kabelgebunden ausgebildet sein.

Insofern umfasst Schritt i) neben einem Erfassen im Sinne von Erzeugen der dreidimensionalen Bildinformation mittels der Erfassungseinheit in erfindungsgemäßen Ausführungen auch ein Erfassen der dreidimensionalen Bildinformation im Sinne von Übertragen mittels der Recheneinheit, die über die Datenleitung und eine Datenschnittstelle mit der Erfassungseinheit in Kommunikation steht.

In besonders bevorzugter Ausführung erfasst die Erfassungseinheit nicht nur eine, sondern gleichzeitig mehrere bewegliche Komponenten der medizinischen Anlage. Die mehreren beweglichen Komponenten können also zeitgleich von einer der 3D-Kameras oder jeweils von einer anderen der mehreren 3D-Kameras erfasst werden. Dies ermöglicht die zeitlich parallele Ausführung des erfindungsgemäßen Bewegungssteuerverfahrens für die mehreren beweglichen Komponenten.

Insbesondere kann mittels der Erfassungseinheit auch ein Patient in der Umgebung der beweglichen Komponente miterfasst und abgebildet werden. Die Erfassungseinheit ist folglich auch ausgebildet, dreidimensionale Bildinformation bezüglich eines Patienten zu erzeugen. Der Patient kann dabei insbesondere auf einer Patientenliege bzw. einem Patientenlagerungstisch der medizinischen Anlage liegen. Andere Patientenpositionen sind aber auch möglich.

Die Erfassungseinheit ermöglicht erfindungsgemäß ein zeitgleiches Erfassen von aktuellen dreidimensionalen Koordinaten der beweglichen Komponente sowie aller anderen starren oder beweglichen Objekte in ihrer Nähe. Im weiteren Verlauf des Verfahrens kann basierend darauf ein Abstand zwischen der bewegten Komponente und wenigstens einem der abgebildeten Objekte ermittelt und auf eine potenzielle Kollision geschlossen werden. Anhand der dreidimensionalen Bildinformation kann auch jederzeit und einfach eine Kontur eines Patienten ermittelt werden.

Ein weiterer Schritt ii) ist auf ein Ermitteln eines ersten Positionsparameters zu der beweglichen Komponente basierend auf der dreidimensionalen Bildinformation mittels der Recheneinheit gerichtet. Der erste Positionsparameter beschreibt eine Lage bzw. Position der beweglichen Komponente in Bezug auf ein vorab definiertes Koordinatensystem. Das Koordinatensystem kann bspw. das Koordinatensystem der 3D-Kamera sein. In anderen Ausführungen kann das Koordinatensystem mit Bezug zu der medizinischen Anlage definiert sein. Mit anderen Worten beschreibt der erste Positionsparameter eine relative Lage der beweglichen Komponente in Bezug zur medizinischen Anlage. Der erste Positionsparameter kann alternativ eine Lage oder Position der beweglichen Komponente zu einem beliebigen, der Recheneinheit aber bekannten Bezugspunkt im Raum angeben.

Das Ermitteln des ersten Positionsparameters umfasst ein Auswerten bzw. Analysieren der dreidimensionalen Bildinformation durch die Recheneinheit. Diese umfasst folglich Mittel bzw. funktionale Einheiten, die ausgebildet sich, die bereitgestellte dreidimensionale Bildinformation zu verarbeiten. Das Ermitteln des ersten Positionsparameters umfasst in Ausführungen einen Schritt zur Objekterkennung in der Bildinformation, um unter den abgebildeten Objekten die bewegliche Einheit zu identifizieren. Bspw. kann zur Identifizierung ein eindeutiger Marker an der beweglichen Einheit angeordnet sein, welcher mittels gängiger Objekterkennungsalgorithmen schnell, einfach und zuverlässig erkennbar ist. Zur Objekterkennung kann die Recheneinheit bspw. an sich bekannte, zuverlässige und robuste Segmentierungsverfahren auf die Bildinformation anwenden, insbesondere um eine Ausdehnung bzw. die Außenkonturen der beweglichen Komponente zu ermitteln. Der erste Positionsparameter kann in Ausführungen die Position eines Mittelpunktes oder Schwerpunktes der beweglichen Komponente beschreiben. In anderen Ausführungen kann der erste Positionsparameter eine Mehrzahl von Eck- oder Konturpositionen der beweglichen Komponente beschreiben. Die konkrete Ausbildung des ersten Positionsparameters richtet sich vorwiegend nach der Gestalt der jeweiligen beweglichen Komponente.

Aufgrund der Komplexität einer 3D-Kamera im Hinblick auf ihre Hardware und Software, kann diese nicht an sich erstfehlersicher ausgeführt werden. Die von der 3D-Kamera bereitgestellte dreidimensionale Bildinformation kann folglich fehlerbehaftet sein, was zu einem fehlerbehafteten ersten Positionsparameter führen kann. Dies könnte zu einem Versagen der Kollisionserkennung führen. Daher muss die Integrität der dreidimensionalen Bildinformation fortlaufend geprüft werden.

Zu diesem Zweck ist ein weiterer Schritt iii) auf ein Erfassen eines zweiten Positionsparameters zu der beweglichen Komponente mittels Recheneinheit gerichtet.

Die Bewegung der medizinischen Anlage, bzw. der beweglichen Komponente wird mittels Recheneinheit der medizinischen Anlage mittels entsprechender Steuersignale gesteuert und überwacht. Die medizinische Anlage bzw. die bewegliche Komponente ist dabei dazu ausgebildet, kontinuierlich Informationen zu erfassen, die einen Vergleich einer aktuellen, tatsächlichen Position oder Lage der beweglichen Komponente mit einer mittels Steuersignal vorgegebenen Soll-Lage bzw. Soll-Position zu vergleichen. Anhand dieser erfassten Informationen kann erfindungsgemäß auch der zweite Positionsparameter für die bewegliche Komponente ermittelt, abgeleitet bzw. direkt erfasst werden.

In bevorzugter Ausführung der medizinischen Anlage umfasst diese eine Sensoreinheit umfassend wenigstens einen Sensor zum Erfassen des zweiten Positionsparameters der beweglichen Komponente. Der wenigstens eine Sensor kann bspw. als an sich bekannter und beliebig ausgebildeter Positionssensor, für mindestens eine Bewegungsachse oder als Winkelmesser für jeweils wenigstens eine Rotations- oder Schwenkachse der beweglichen Komponente ausgeführt sein. Andere Ausführungen sind ebenfalls möglich. Typischerweise ist der wenigstens eine Sensor im Antriebsstrang oder an der beweglichen Komponente bereits zum Zweck der Steuerung/Regelung der Komponentenbewegung vorhanden und erfasst auch bereits die oben beschriebenen Größen, sodass dieser aufwandsarm im Sinne der Erfindung genutzt werden kann. Recheneinheit und Sensoreinheit stehen erfindungsgemäß bevorzugt über eine Datenverbindung in Kommunikation, sodass die Sensordaten zur Weiterverarbeitung an die Recheneinheit übertragen werden können. Insofern umfasst Schritt iii) neben dem Erfassen des zweiten Positionsparameters mittels der Recheneinheit zumindest in Ausführungen auch das Erfassen bzw. das Messen des zweiten Positionsparameters mittels der Sensoreinheit.

Der erste und/oder der zweite Positionsparameter der beweglichen Komponente sind in vorteilhafter Weiterbildung der Erfindung in gleicher Form ausgebildet, um sie im weiteren Verlauf des Verfahrens besonders einfach miteinander vergleichen zu können.

Das Erfassen bzw. Ermitteln des ersten und zweiten Positionsparameters zu der beweglichen Komponente ermöglicht nun eine einfache Überprüfung der Funktionalität der 3D-Kamera. Da der zweite Positionsparameter unabhängig von der 3D-Kamera erfasst bzw. erzeugt wird, kann das erfindungsgemäße Verfahren erstfehlersicher ausgeführt werden.

Entsprechend ist ein weiterer Schritt iv) des erfindungsgemäßen Verfahrens auf ein Vergleichen des ersten und des zweiten Positionsparameters mittels Recheneinheit gerichtet. Mit anderen Worten umfasst Schritt iv) eine Gegenüberstellung des ersten und des zweiten Positionsparameters. Der Parametervergleich kann eine Übereinstimmung oder eine Abweichung der Parameter voneinander aufzeigen. In Abhängigkeit des Vergleichsergebnisses werden weitere Schritte des Verfahrens ausgeführt oder nicht. Der Vergleich wird durch die Recheneinheit ausgeführt, diese ist entsprechend ausgebildet, jeweilige Werte für die Positionsparameter zu erfassen und im Sinne eines Vergleichs weiterzuverarbeiten.

Eine Übereinstimmung wird erkannt, wenn der erste und der zweite Positionsparameter lediglich im Rahmen eines ersten, vorab festgelegten Schwellwertes voneinander abweichen. Der erste Schwellwert kann insbesondere spezifisch für die jeweils betrachtete bewegliche Komponente sein. In Ausführungen der Erfindung sind die zulässigen Abweichungen zwischen erstem und zweitem Positionsparameter kleiner bei einer beweglichen Komponente, die in direkter Nähe bzw. sehr nahe am Patienten bewegt wird, bspw. einem Strahlenschutzschirm oder dergleichen. Der erste Schwellwert kann alternativ oder zusätzlich auch betriebszustandsbedingte Toleranzen des Antriebsstrangs der beweglichen Komponente berücksichtigen, sprich, veränderlich bzw. adaptiv ausgebildet sein. Bspw. kann der erste Schwellwert größer ausgebildet sein, wenn die Betriebstemperatur höher ist. Bspw. kann für Temperaturveränderungen eine Look-Up-Tabelle für den ersten Schwellwert abrufbar hinterlegt sein, wobei dass für einen Vergleich des ersten und des zweiten Positionsparameters auch ein aktueller Temperaturwert nahe der beweglichen Komponente erfasst wird.

Ein weiterer Schritt v) ist auf ein Erzeugen eines Bewegungssteuersignals für die bewegliche Komponente basierend auf der dreidimensionalen Bildinformation mittels Recheneinheit gerichtet, sofern die Abweichung zwischen erstem und zweitem Positionsparameter den ersten Schwellwert unterschreitet.

Hält die Abweichung den ersten Schwellwert ein, sprich, stimmen erster und zweiter Positionsparameter im Rahmen des vorgegebenen Toleranzmaßes überein, ist erfindungsgemäß die korrekte Funktionalität der 3D-Kamera bestätigt. Das erfindungsgemäße Verfahren geht folglich davon aus, dass die mittels der 3D-Kamera erfasste Bildinformation die tatsächliche Lage/Position der beweglichen Komponente, insbesondere die relative Lage der beweglichen Komponente in Bezug zu weiteren in der Bildinformation abgebildeten Objekten in der Umgebung der beweglichen Komponente korrekt darstellt. Insofern sieht die Erfindung für diesen Fall die Erzeugung eines Bewegungssteuersignals vor, wobei die Erzeugung des Steuersignals eine weitere Auswertung/Analyse/Verarbeitung der dreidimensionalen Bildinformation mittels der Recheneinheit umfasst.

Stellt sich bei dem Vergleich des ersten und des zweiten Positionsparameters heraus, dass die Abweichung oberhalb des ersten Schwellwertes liegt, wird erfindungsgemäß von einer Fehlfunktion der 3D-Kamera und davon ausgegangen, dass die Ergebnisse einer weiteren Analyse/Auswertung/Verarbeitung der dreidimensionalen Bildinformation zur Erzeugung eines Bewegungssteuersignals ungeeignet, da fehlerbehaftet sind. Ein weiterer, zu Schritt v) optionaler Schritt vi) ist folglich auf ein Erzeugen eines Fehlersignals mittels Recheneinheit gerichtet, sofern die Abweichung zwischen erstem und zweitem Positionsparameter den ersten Schwellwert überschreitet.

Ein weiterer Schritt vii) ist auf ein Ausführen des Bewegungssteuersignals durch die bewegliche Komponente oder des Fehlersignals durch die Recheneinheit gerichtet.

Ein erzeugtes Bewegungssteuersignal umfasst erfindungsgemäß wenigstens einen Steuerbefehl für eine Antriebseinheit der beweglichen Komponente und/oder der medizinischen Anlage, wobei die Antriebseinheit dazu ausgebildet ist, die gewünschte Bewegung der beweglichen Komponente zu bewirken. Ein Steuerbefehl kann in Ausführungen, Richtungsvorgaben, Geschwindigkeit- oder Beschleunigungsvorgaben, Winkelvorgaben, Zielpositionen, Zielstellungen oder dergleichen umfassen. Ein Steuerbefehl wird erfindungsgemäß anhand der mit der 3D-Kamera erfassten Bildinformation erzeugt.

In bevorzugter Ausführung des Bewegungssteuerverfahrens sieht das Fehlersignal eine Warnsignalausgabe und/oder das Aktivieren eines Kollisionsvermeidungsverfahrens vor. Mit anderen Worten steuert das Fehlersignal eine optische und/oder akustische Ausgabe eines Warnsignals an einen Benutzer der medizinischen Anlage. Das Warnsignal kennzeichnet insbesondere die Fehlfunktion der 3D-Kamera. Das Warnsignal kann in Ausführungen auch eine Veränderung des Bewegungsverhaltens der beweglichen Komponente anzeigen. Bspw. kann das Warnsignal einen Bewegungsstopp der beweglichen Komponente anzeigen oder ankündigen. Das Warnsignal kann auch anzeigen, dass eine bisherige erfindungsgemäße Bewegungssteuerung in eine Bewegungssteuerung entsprechend einem manuellen Bewegungsbetrieb oder einem klassischen Kollisionsvermeidungsalgorithmus übergeht. Entsprechend kann das erzeugte Fehlersignal alternativ oder zusätzlich ein Steuersignal umfassen, welches ausgebildet ist, die Recheneinheit in einen anderen Betriebsmodus entsprechend einem manuellen Bewegungsbetrieb oder entsprechend einem klassischen Kollisionsvermeidungsalgorithmus, welche jeweils von der erfindungsgemäßen Bewegungssteuerung insofern abweichen, als die mittels der 3D-Kamera erfasste dreidimensionale Bildinformation von der Erzeugung von Bewegungssteuersignalen für die bewegliche Komponente ausgeschlossen wird.

Die von der medizinischen Anlage umfasste Recheneinheit ist ausgebildet, die einzelnen Schritte des erfindungsgemäßen Verfahrens auszuführen. Insbesondere ist die Recheneinheit ausgebildet, eine Objekterkennung auf den dreidimensionalen Bilddaten auszuführen und daraus den ersten Positionsparameter abzuleiten. Die Recheneinheit ist weiter ausgebildet, den ersten und den zweiten Positionsparameter miteinander zu verrechnen, insbesondere zu vergleichen und basierend auf dem Vergleich Steuersignale zu erzeugen.

Die Recheneinheit kann als eigenständige Recheneinheit, insbesondere als Recheneinheit der medizinischen Anlage oder als Teil eines Rechensystems, bspw. einer zentralen Steuerungseinheit eines Krankenhauses oder dergleichen ausgebildet sein. Die Recheneinheit kann eine Speichereinheit umfassen oder mit dieser in Datenkommunikation stehen, in der abrufbar bspw. Look-Up Tabellen für den ersten Schwellwert oder eine Programmroutine umfassend eine Abfolge von Prozessschritten zur erfindungsgemäßen Ermittlung von Bewegungssteuersignalen für die bewegliche Komponente basierend auf den dreidimensionalen Bildinformationen und/oder eine Programmroutine für einen klassischen Kollisionsvermeidungsalgorithmus oder ähnliches hinterlegt sind.

Die Recheneinheit kann in Ausführungen der Erfindung als eine oder mehrere zentrale und/oder dezentrale Rechenmodule ausgebildet sein. Die Rechenmodule können jeweils einen oder mehrere Prozessoren aufweisen. Ein Prozessor kann als zentrale Verarbeitungseinheit (CPU/GPU) ausgebildet sein. Alternativ kann die Recheneinheit als lokaler oder Cloud-basierter Verarbeitungsserver implementiert sein. Ferner kann die Recheneinheit ein oder mehrere virtuelle Maschinen umfassen.

Die Recheneinheit ist weiter zur Datenkommunikation, insbesondere mit der Speichereinheit, der Sensoreinheit, der 3D-Kamera, der Antriebseinheit der beweglichen Komponente o.ä. ausgebildet. Die Recheneinheit umfasst zu diesem Zweck eine Schnittstelle, die allgemein zum Datenaustausch ausgebildet sein kann. Die Schnittstelle kann insofern in Form von einer oder mehreren einzelnen Datenschnittstellen implementiert sein, welche ein Hardware- und/oder Software-Interface, einen Daten-Bus, bspw. einen PCI-Bus, eine USB-Schnittstelle, eine Fire-Wire-Schnittstelle, eine ZigBee- oder eine Bluetooth-Schnittstelle aufweisen können. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle oder eine Wireless LAN-Schnittstelle (WLAN oder Wi-Fi) aufweisen.

In bevorzugter Ausführung umfasst Schritt v), das Erzeugen des Bewegungssteuersignals, einen Abstand zwischen der beweglichen Komponente und einem Hindernis in der Umgebung der beweglichen Komponente anhand der dreidimensionalen Bildinformation zu bestimmen. Erfindungsgemäß wird neben der beweglichen Komponente selbst also wenigstens ein weiteres, von der beweglichen Komponente unabhängiges Objekt in der dreidimensionalen Bildinformation erkannt bzw. als Hindernis identifiziert. Auch für das wenigstens eine weitere Objekt wird anhand der umfassten 3D-Information eine Position für das Objekt und im Folgenden ein Abstand zwischen beweglicher Komponente und Objekt ermittelt. Das erfindungsgemäße Verfahren kann auch hier zur Objekterfassung einen Marker an dem Objekt abbilden und/oder dieses bzw. das Objekt mittels gängiger Objekterkennungsalgorithmen schnell, einfach und zuverlässig erkennen.

Zur Objekterkennung kann die Recheneinheit auch hier an sich bekannte, zuverlässige und robuste Segmentierungsverfahren auf die Bildinformation anwenden, insbesondere um eine Ausdehnung bzw. die Außenkonturen des wenigstens einen Objekts zu ermitteln. Bevorzugt werden alle in der dreidimensionalen Bildinformation dargestellten Objekte erkannt. Für jedes Objekt kann gleich dem ersten Positionsparameter die Position des Objektes als die Position eines Mittelpunktes oder Schwerpunktes des Objektes beschrieben werden. Alternativ oder zusätzlich kann die Position eines Objektes auch Informationen zu dessen Ausdehnung, bspw. die Position von Eck- oder Konturpunkten des Objektes umfassen. Der Abstand zwischen beweglicher Komponente und jedem erkannten Objekt kann in Ausführungen dazu dienen, ein Objekt als Hindernis zu klassifizieren. Bspw. wird ein Objekt als Hindernis klassifiziert, wenn ein vorab definiertes Abstandslimit unterschritten wird. Das definierte Abstandslimit kann insbesondere von der Art des erkannten Objektes oder einer Absolutposition der beweglichen Komponente abhängen und dementsprechend jeweils unterschiedlich ausfallen.

In Ausführungen der erfindungsgemäßen Programmroutine kann daher weiter vorgesehen sein, die Art des Objektes anhand der dreidimensionalen Bildinformation, insbesondere der abgeleiteten Konturpunkte und darauf basierend das vorab definierte Abstandslimit zu ermitteln.

In anderen Ausführungen kann ein in der dreidimensionalen Bildinformation erkanntes Objekt in Abhängigkeit seiner Art als Hindernis klassifiziert werden. In wieder anderen Ausführungen kann jedes in der dreidimensionalen Bildinformation erkannte Objekt als Hindernis klassifiziert werden, da von jedem Objekt grundsätzlich ein Hindernis für die bewegliche Komponente darstellen kann.

Diese erfindungsgemäße Vorgehensweise geht nach einem Vergleich des ersten und des zweiten Positionsparameters und Einhalten des ersten Schwellwerts davon aus, dass die aus der dreidimensionalen Bildinformation ableitbaren Positions- und Abstandsdaten in Bezug auf weitere abgebildete Objekt ebenfalls korrekt und realitätsgetreu und damit für eine Bewegungsplanung bzw. Bewegungssteuerung verwendbar sind.

Der so ermittelte Abstand zwischen wenigstens einem Objekt und der beweglichen Komponente wird in Ausführungen entsprechend zur Erzeugung des Bewegungssteuersignals berücksichtigt. Mit anderen Worten basiert das Bewegungssteuersignal auf dem ermittelten Abstand.

Ein Objekt, bzw. ein Hindernis kann in Ausführungen der Erfindung als starres oder bewegtes Hindernis ausgebildet sein. Ein starres, also unbewegliches Hindernis ist bspw. ein bodenmontiertes Stativ der medizinischen Anlage oder eine ebenfalls bodenmontierte Gantry einer weiteren medizinischen Anlage, in deren Umgebung die bewegliche Komponente sich bewegt. Ein bewegtes Hindernis ist bspw. eine Person des medizinischen Personals, welches die medizinische Anlage bedient, ein Patient oder beliebiges anderes, fahr- oder bewegbares medizinisches Gerät in der Umgebung der beweglichen Komponente.

Ein Objekt bzw. Hindernis ist in weiteren Ausführungen als eine nicht zu der medizinischen Anlage gehörige Einheit ausgebildet. Das Hindernis steht also nicht direkt oder indirekt physisch mit der beweglichen Komponente in Verbindung. Bspw. ist das Objekt als eine weitere in der Umgebung der beweglichen Komponente befindliche medizinische Anlage ausgebildet. Alternativ dazu ist das Objekt bzw. Hindernis direkt oder indirekt physisch mit der beweglichen Komponente verbunden, ist also ebenfalls Teil oder Komponente der medizinische Anlage, bspw. ein Patientenlagerungstisch, ein C-Bogen, eine Röntgenstrahlenquelle oder dergleichen.

Umfasst eine medizinische Anlage mehrere bewegliche Komponenten und werden diese zeitgleich mittels der 3D-Kamera erfasst, kann für jede der beweglichen Komponenten die abgebildete Umgebung im Hinblick auf Objekte bzw. Hindernisse und deren Abstände zur beweglichen Komponente hin analysiert werden. Dabei ist insbesondere zu beachten, dass eine erste bewegliche Komponente ein Hindernis für eine zweite bewegliche Komponente darstellen kann oder umgekehrt.

In einer besonders bevorzugten Ausführung der Erfindung, in welcher die bewegliche Komponente ein Patientenlagerungstisch ist, auf welchem ein Patient positioniert ist, erfolgt das Bestimmen des Abstands zwischen der beweglichen Komponente und dem Hindernis, indem auch ein Abstand zwischen dem Patienten und dem Hindernis basierend auf der dreidimensionalen Bildinformation bestimmt wird. Anders ausgedrückt wird in dieser Ausführung der Patient als ein weiteres Objekt in den Bilddaten erkannt. Erfolgt eine Identifikation, bspw. anhand der ermittelten Konturpunkte des Patienten, als Patient, so wird dieser jedoch im weiteren Verlauf des Verfahrens der beweglichen Komponente zugerechnet und nicht als weiteres Objekt bzw. Hindernis klassifiziert. Insofern ist vorgesehen, eine gemeinsame Positionsinformation für Patient und Patientenlagerungstisch zu erfassen. Dabei wird erfindungsgemäß davon ausgegangen, dass der Patient still und unbeweglich auf dem Patientenlagerungstisch verharrt. Bspw. kann vorgesehen sein, für Patientenlagerungstisch und Patient aus den erfassten Konturpunkten eine gemeinsame Kontur zu ermitteln. Alternativ oder zusätzlich kann daraus ein gemeinsamer Mittel- oder Schwerpunkt errechnet werden. In dieser Ausführung wird folglich neben dem Patienten wenigstens ein weiteres Objekt in der dreidimensionalen Bildinformation erfasst und als Hindernis klassifiziert. Es wird weiter ein Abstand zwischen dem Hindernis und der gemeinsamen Positionsinformation für Patientenlagerungstisch und Patient abgeleitet.

In bevorzugten Ausführungen des erfindungsgemäßen Verfahrens sieht das derart ermittelte Bewegungssteuersignal eine Geschwindigkeitserhöhung für die bewegliche Komponente vor, wenn der Abstand zwischen beweglicher Komponente und Hindernis einen ersten Abstandsschwellwert überschreitet. Alternativ sieht das Bewegungssteuersignal eine Geschwindigkeitsreduktion für die bewegliche Komponente vor, wenn der Abstand zwischen beweglicher Komponente und Hindernis den ersten Abstandsschwellwert unterschreitet. Der erste Abstandsschwellwert kann bspw. der Größe einer ersten Sicherheitszone entsprechen, von welcher die bewegliche Komponente umgeben ist. Der erste Abstandsschwellwert kann ebenfalls für eine jede bewegliche Komponente spezifisch ausfallen und abrufbar in der Speichereinheit hinterlegt sein.

Mit anderen Worten ermöglicht die erfindungsgemäße Programmroutine für den Fall des Unterschreitens des ersten Abstandsschwellwerts eine sichere Vorhersage, dass eine Kollision kurz bevorsteht. Für den Fall, dass sich kein Hindernis im Bereich der ersten Sicherheitszone befindet, kann erfindungsgemäß die Bewegungsgeschwindigkeit über bislang zulässige Geschwindigkeiten erhöht und damit der Bewegungsablauf sicher beschleunigt werden. Entsprechend initiiert die Recheneinheit über die Erzeugung des Bewegungssteuersignals dynamisch Kollisionsvermeidungsmaßnahmen, bspw. die Reduktion der Geschwindigkeit, oder beschleunigt die Bewegung, wenn keine Kollisionsgefahr erkennbar ist. Andere Kollisionsvermeidungsmaßnahmen sind natürlich ebenfalls möglich und erfindungsgemäß vorgesehen. Bspw. kann das Bewegungssteuersignal in Ausführungen eine Richtungsänderung für die bewegliche Komponente vorsehen, um eine Kollision zu vermeiden, wenn der Abstand zwischen beweglicher Komponente und Hindernis einen zweiten Abstandsschwellwert unterschreitet. Der zweite Abstandsschwellwert ist dabei kleiner als der erste Abstandsschwellwert und entspricht einer zweiten, kleineren Sicherheitszone. Verletzt ein Hindernis die zweite Sicherheitszone der beweglichen Einheit, so initiiert die Recheneinheit über das Bewegungssteuersignal mittels der vorgesehenen Richtungsänderung, insbesondere einer Richtungsumkehr ein Ausweichmanöver. Alternativ kann das Bewegungssteuersignal, bspw. zum Auslösen einer Bremsaktion der Antriebseinheit, auch einen Bewegungsstopp der beweglichen Komponente auslösen.

Zusätzlich kann in Ausführungen auch in diesem Fall das Bewegungssteuersignal eine parallele Ausgabe eines optischen oder akustischen Warnsignals vorsehen und steuern. Dieses kann bspw. in Lautstärke und/oder Farbe an den ermittelten Abstand angepasst werden.

Die erfindungsgemäßen Verfahrensschritte i) bis vii) werden bevorzugt während einer Bewegung der beweglichen Komponente ausgeführt, mit dem Zweck, diese Bewegung abzusichern und im Bedarfsfall die geplante Bewegungsroute anpassen zu können. Selbstredend kann die erfindungsgemäße Programmroutine aber auch ablaufen, wenn sich die bewegliche Komponente in Ruhe, also in einem unbewegten Zustand befindet. In dieser Ausführung werden Bewegungen bewegter Hindernisse in der Umgebung überwacht und bei Kollisionsgefahr eine Bewegung der beweglichen Komponente und/oder ein Warnsignal für Bedienpersonal ausgelöst, um die Kollision zu vermeiden.

Da eine Bewegung der beweglichen Komponente sich über einen gewissen Zeitraum hinweg erstrecken kann, wobei der gesamte Zeitraum in Bezug auf Kollisionen überwacht werden muss. Entsprechend werden die Schritte der Programmroutine in bevorzugter Ausführung mehrfach, also wiederholt ausgeführt. Besonders vorteilhaft ist eine Wiederholrate von 15 Hz entsprechend einer Bildrate der 3D-Kamera. Derart stellt das erfindungsgemäße Verfahren eine Quasi-kontinuierliche Kollisionsüberwachung für die bewegliche Komponente bereit, sodass abstände und insbesondere Verletzungen von Sicherheitszonen quasi instantan, also ohne merklichen Zeitverzug detektiert werden und Gegenmaßnahmen initiiert werden können.

Durch die gleichzeitige Erfassung der dreidimensionalen Positionsinformation der beweglichen Komponente über den Verlauf seiner Bewegung sowie weiterer Objekte in der Nähe der beweglichen Komponente (wie bspw. weitere medizinische Geräte, ein Patient, andere Personen), kann erfindungsgemäß vorteilhaft einfach und jederzeit errechnet werden, wie weit die bewegliche Komponente von einem Hindernis entfernt ist. Da der ermittelte Abstand als analoger Wert vor einer eigentlichen Kollision verfügbar ist, kann das erfindungsgemäße Verfahren über die Erzeugung eines entsprechenden Steuersignals dafür sorgen, dass die Geschwindigkeit der beweglichen Komponente reduziert wird, was die Kräfte bei einer Kollision signifikant reduzieren und einen möglichen Schaden minimieren kann. Bspw. kann die Reduktion der Geschwindigkeit dabei proportional zu einer Abstandsverringerung erfolgen. Außerdem kann der Bediener vor einer drohenden Kollision, z.B. durch einen Warnton, gewarnt werden.

Der erfindungsgemäße Abgleich zwischen erstem und zweiten Positionsparameter prüft in regelmäßigen, kurzen zeitlichen Abständen, also quasi permanent bzw. fortlaufend, ob die mittels 3D-Kamera erfasste Position der beweglichen Komponente mit der tatsächlichen Position derselben übereinstimmt. Darüber wird die korrekte, verlässliche Funktion der 3D-Kamera verifiziert. Stimmen ersten und zweiter Positionsparameter überein, wird bei der weiteren Bewegungsplanung bzw. Bewegungssteuerung davon ausgegangen, dass die 3D-Kamera fehlerfrei funktioniert und für die Kollisionserkennung verwendet werden kann. Weichen erster und zweiter Positionsparameter jedoch voneinander ab, so wird ein erfindungsgemäß ein Fehler gemeldet und eine Sicherheitsreaktion ausgelöst (z.B. ein Bewegungstop oder eine Bewegung mit reduzierter Geschwindigkeit). Eine erfindungsgemäße Sicherheitsreaktion kann einer heutigen Sicherheitsreaktion entsprechen, die bspw. ausgelöst wird, wenn ein Funktionsfehler in einem Kollisionssensor festgestellt wird.

Um die Erfordernisse der Erstfehlersicherheit im medizintechnischen Bereich zu erfüllen, ist in vorteilhaften Ausführungen des Bewegungssteuerverfahrens vorgesehen, dass die Schritte ii) bis vi) redundant und unabhängig voneinander ausgeführt werden. Die Redundanz kann dabei dadurch erreicht werden, dass die Rechenschritte mehrfach ausgeführt werden. Dies ist jedoch nachteilig im Hinblick auf die Prozessdauer. Um die Unabhängigkeit der beiden Berechnungspfade zu erreichen, werden die Prozessschritte bevorzugt auf zwei voneinander unabhängigen Rechenmodulen der Recheneinheit ausgeführt. Dabei arbeiten die beiden Rechenmodule quasi zeitgleich nebeneinander. Nur bei Übereinstimmung der Rechenergebnisse der beiden Rechenmodule werden diese zur Weiterverarbeitung zur Erzeugung eines Steuersignals herangezogen. Damit werden insbesondere auch die computerimplementierten Verfahrensschritte erstfehlersicher ausgeführt. Insofern realisiert die Erfindung in dieser Ausführung eine Steuerarchitektur umfassend einen Steuerpfad (C-Pfad) sowie einen Schutz-Pfad (P-Pfad). Die einzelnen Rechenmodule können bspw. als zwei voneinander unabhängige CPUs ausgebildet sein.

Heute bekannte Kollisionssensoren zur Detektion einer erfolgten Kollision liefern ein binäres Kollisionssignal. Die Erfindung realisiert über die Ausbildung eines Schutzpfades zusätzlich zu dem eigentlichen Steuerpfad mittels der leistungsstarken Komponenten der Recheneinheit eine sichere, zyklische Überwachung der Funktionalität der 3D-Kamera anhand der erfassten Position der beweglichen Komponente. Gleichzeitig werden Abstände der beweglichen Komponente zu ebenfalls erfassten Hindernissen berechnet. Werden vorab definierten Sicherheitszonen verletzt bzw. Abstände unterschritten, wird erfindungsgemäß über einen sicheren Ausgang bzw. ein Sicherheitsprotokoll eine sich anbahnende Kollision gemeldet. Ist der ermittelte Abstand bereits null oder nahe null, so wird über einen sicheren Ausgang bzw. das Sicherheitsprotokoll eine Kollision gemeldet. Durch die ebenfalls erstfehlersichere Abstandmessung zum nächsten Hindernis kann das erfindungsgemäße Verfahren ein Frei- bzw. Bewegungsraum bestimmen, in dem eine kollisionsfreie Bewegung möglich, sich die bewegliche Komponente weiterbewegen kann. Eine Bewegung in diesem Bewegungsraum ist dann ohne Aktivierung eines klassischen Kollisionsvermeidungsalgorithmus möglich.

Die Berechnung z.B. mit der typischen VGA-Auflösung von 15 fps/Hz kann bspw. auf Recheneinheiten in Form moderner embedded Applikationsprozessoren mit mehreren CPU-Kernen oder einer GPU durchgeführt werden. Die zweikanalige Steuerarchitektur mit Steuer- und Schutzpfad ist technisch deutlich leichter zu realisieren als einen Prozessrechner an sich erstfehlersicher zu realisieren. Denn dies stieße mit der Medizingerätenorm IEC60601 an Probleme, die eine absolute Sicherheit (= kein Fehler) fordert und Fehlerwahrscheinlichkeiten, wie in der vergleichbaren Industrienorm IEC61508 zulässig, ausschließt. Die Medizingerätenorm IEC60601 erlaubt eine zweikanalige Sicherheitsarchitektur. Das Steuer-Kanal-Rechenmodul ist als normaler Computer oder embedded Computer ausgebildet, an den keine besonderen Sicherheitsanforderungen gestellt werden. Bei fehlerfreier Funktion, führt dieser auch die Kollisionsreaktion (bspw. Geschwindigkeit verringern, anhalten) aus. Im Falle eines Fehlers, steht das Schutzpfad-Rechenmodul bereit und übernimmt die Funktion des Steuer-Pfad-Rechenmoduls. Die Zweikanaligkeit reduziert das Maß der Sicherheitsanforderungen an das Steuer-Pfad-Rechenmodul. Ein dort auftretender Fehler muss nicht mehr nach der Prozessfehlertoleranzzeit (typischerweise 100 ms), sondern erst nach der Mehrfachfehlereintrittszeit (typischerweise 1 Tag) erkannt werden. Bleibt die Schutzreaktion des Steuer-Pfad-Rechenmoduls aus, übernimmt das Schutz-Pfad-Rechenmodul mit der vorhandenen P-Pfad-Infrastruktur die nötige Schutzreaktion.

Wie eingangs beschrieben, kann erfindungsgemäß zur Bewegungssteuerung aber auch ein klassischer Kollisionsvermeidungsalgorithmus zum Einsatz kommen. Einerseits wird dieser in Ausführungen erfindungsgemäß aktiviert, wenn die Abweichung zwischen erstem und zweitem Positionsparameter größer ist als der ersten Schwellwert, die dreidimensionale Bildinformation also nicht ausreichend verlässlich ist. Dabei sind dann die Hindernisse, die von dem Algorithmus bei der Pfadplanung berücksichtigt werden können, beschränkt auf vorab bekannte Objekte, insbesondere weitere Komponenten der medizinischen Anlage.

In einer weiteren Ausführungsform des erfindungsgemäßen Verfahrens, in welcher die Abweichung zwischen erstem und zweitem Positionsparameter den ersten Schwellwert einhält, die dreidimensionale Bildinformation folglich verlässliche Positionsinformation zu weiteren Objekten liefert, wird in Schritt v) das Bewegungssteuersignal unter Anwendung eines Kollisionsvermeidungsverfahrens erzeugt, welches den ermittelten Abstand zwischen beweglicher Komponente und Hindernis berücksichtigt. Mit anderen Worten wird erfindungsgemäß die berücksichtige Objektmenge des Kollisionsvermeidungsalgorithmus anhand der dreidimensionalen Bildinformation, konkret anhand der daraus abgeleiteten Positions- und Abstandsinformation zu den weiteren Objekten in der Nähe der beweglichen Komponente ergänzt. Derart ermöglicht die Erfindung, diese weiteren, dem Kollisionsvermeidungsalgorithmus bis dahin unbekannten Objekte bei der Bewegungssteuerung zu berücksichtigen.

Die Erfindung betrifft in einem weiteren Aspekt ein Computerprogrammprodukt, das ein Programm umfasst und direkt in einen Speicher einer insbesondere programmierbaren Recheneinheit ladbar ist und Programmmittel bzw. Programmabschnitte, bspw. Bibliotheken und Hilfsfunktionen, aufweist, um ein Bewegungssteuerverfahren gemäß den vorgenannten Implementierungen/Aspekten auszuführen, wenn das Computerprogrammprodukt in der Recheneinheit ausgeführt wird.

Ferner betrifft die Erfindung in einem weiteren Aspekt ein computerlesbares Medium, auf welchem einlesbare und ausführbare Programmabschnitte gespeichert sind, um alle Schritte eines Verfahrens zur Bewegungssteuerung gemäß den vorgenannten Implementierungen/Aspekten auszuführen, wenn die Programmabschnitte von einer Recheneinheit ausgeführt werden.

Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Recheneinheit zu laden ist. Durch das Computerprogrammprodukt können die erfindungsgemäßen Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, einen entsprechenden Prozessor oder eine entsprechende Logikeinheit aufweisen, sodass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor der jeweiligen Recheneinheit geladen werden kann, der mit der Recheneinheit direkt verbunden oder als Teil der Recheneinheit ausgebildet sein kann. Weiterhin können Steuerinformationen des Computerprogrammprodukts auf einem computerlesbaren Speichermedium gespeichert sein. Die Steuerinformationen des computerlesbaren Speichermediums können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemä-ßen Ausführungsformen/Aspekte der vorab beschriebenen Verfahren durchgeführt werden. So kann die Erfindung auch von dem besagten computerlesbaren Medium und/oder dem besagten computerlesbaren Speichermedium ausgehen. Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet. Die Figuren sind nicht maßstäblich. Es zeigen:
- FIG 1: ein schematisches Ablaufdiagramm eines Bewegungssteuerverfahrens für eine medizinische Anlage gemäß einer Ausführungsform,
- FIG 2: eine Detailansicht eines Ablaufdiagramms eines Bewegungssteuerverfahrens gemäß einer weiteren Ausführungsform,
- FIG 3: eine Detailansicht eines Ablaufdiagramms eines Bewegungssteuerverfahrens gemäß einer weiteren Ausführungsform,
- FIG 4: eine Detailansicht eines Ablaufdiagramms eines Bewegungssteuerverfahrens gemäß einer weiteren Ausführungsform,
- FIG 5: eine weitere Detailansicht eines Ablaufdiagramms eines Bewegungssteuerverfahrens gemäß einer weiteren Ausführungsform, und
- FIG 6: eine Ansicht einer medizinischen Anlage in Form einer C-Bogen-Röntgenanlage, kurz einer AngiographieAnlage gemäß einer Ausführungsform der vorliegenden Erfindung mit einer Erfassungseinheit umfassend zwei 3D-Kameras.

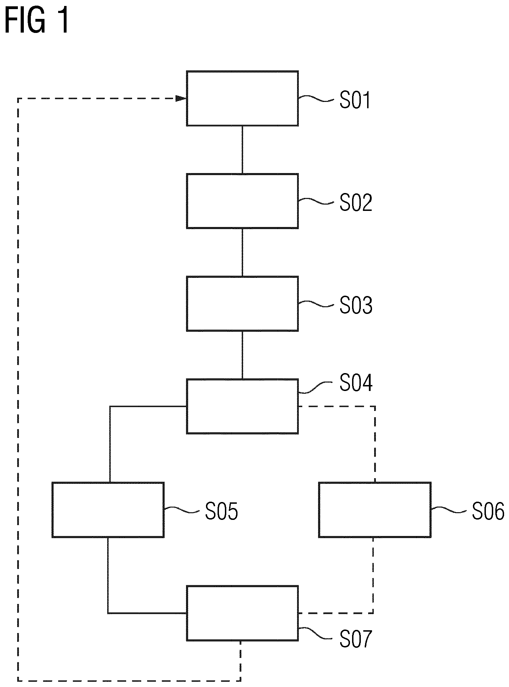

**Figur 1** zeigt ein schematisches Ablaufdiagramm eines Bewegungssteuerverfahrens für eine medizinische Anlage 100 gemäß einer Ausführungsform.

Das Verfahren zur Bewegungssteuerung wird überwiegend oder vollständig durch die Recheneinheit 30 der medizinischen Anlage 100 ausgeführt und umfasst eine Vielzahl von Schritten.

Die Reihenfolge der Schritte ist nicht durch deren Aufzählungsreihenfolge festgelegt. Insbesondere können einzelne Schritte zeitgleich bzw. parallel zueinander ausgeführt werden.

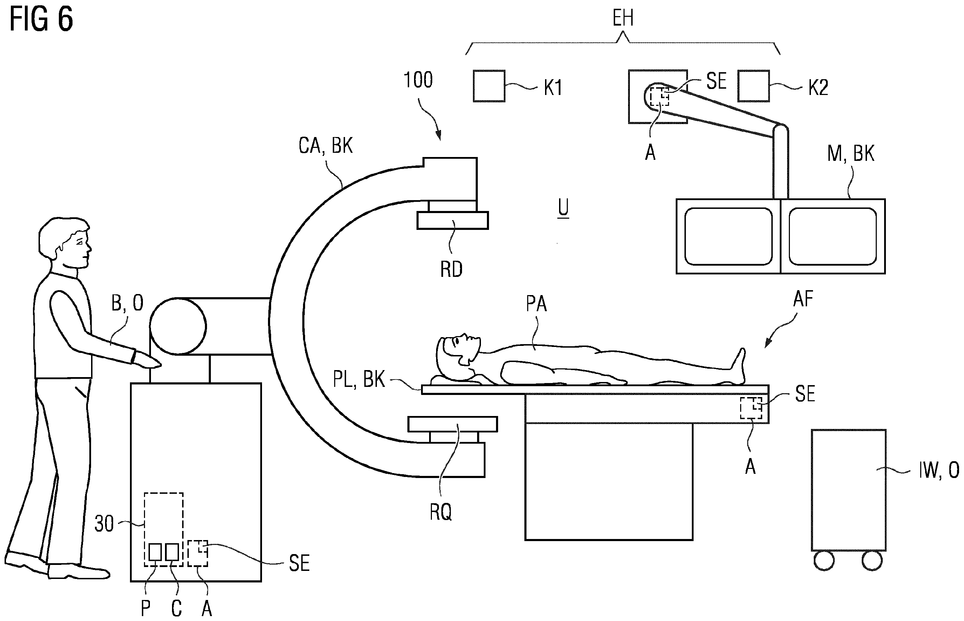

Ein erster Schritt S01 umfasst ein Erfassen einer dreidimensionalen Bildinformation 3DB von einer beweglichen Komponente BK und ihrer Umgebung U mittels einer Erfassungseinheit EH. Die einzelnen Komponenten werden mit Bezug zu Figur 6 noch genauer beschrieben. Das Erfassen schließt das Messen bzw. das Detektieren der dreidimensionalen Bildinformation 3DB mittels Erfassungseinheit EH, aber auch das Empfangen der dreidimensionalen Bildinformation 3DB durch die Recheneinheit 30 über einen Datenkommunikationskanal ein.

Die dreidimensionale Bildinformation 3DB umfasst dreidimensionale Positionsinformationen zu der beweglichen Komponente BK sowie weiterer, in der Umgebung U derselben befindlicher Objekte, die im weiteren Verlauf des Verfahren vorteilhaft ausgewertet und verwendet werden kann.

In einem Schritt S02 erfolgt ein Ermitteln eines ersten Positionsparameters PP1 zu der beweglichen Komponente BK basierend auf der dreidimensionalen Bildinformation 3DB mittels Recheneinheit 30. Der erste Positionsparameter PP1 umfasst Angaben zu einer Position der beweglichen Komponente BK, bspw. in Bezug auf ein Koordinatensystem der Erfassungseinheit EH. Das Ableiten des ersten Positionsparameters PP1 ist erfindungsgemäß besonders leicht möglich, da die dreidimensionale Bildinformation 3DB neben zweidimensionaler Bildinformation auch Tiefeninformationen umfasst. Das Ermitteln des ersten Positionsparameters PP1 kann an sich bekannte Mittel oder Methoden der Objekterkennung umfassen, bspw. den Einsatz eines Markers an der beweglichen Komponente BK und/oder Segmentierungsalgorithmen zur Erfassung von Kontur bspw. dem Volumen, Mittel- oder Schwerpunkten der beweglichen Komponente BK.

In einem Schritt S03 erfolgt ein Erfassen eines zweiten Positionsparameters PP2 zu der beweglichen Komponente BK. Das Erfassen des zweiten Positionsparameters PP2 umfasst bevorzugt das Erfassen desselben mittels einer Sensoreinheit SE. Die Sensoreinheit SE ist ausgebildet, Sensordaten bevorzugt aus dem Antriebsstrang der beweglichen Komponente BK in Form von Positions- oder Winkeldaten zu liefern. Schritt S02 umfasst auch das Empfangen des zweiten Positionsparameters PP2 mittels der Recheneinheit 30 über einen entsprechenden Kommunikationskanal. Auch der zweite Positionsparameter PP2 umfasst Angaben über eine aktuelle Position der beweglichen Komponente BK. Erster und zweiter Positionsparameter PP1, PP2 sind dadurch gekennzeichnet, dass sie mittels zweier unabhängiger Messmittel erfasst werden. Erster und zweiter Positionsparameter PP1, PP2 umfassen bevorzugt die gleiche Positionsinformation, sodass ein Abgleich beider Positionsparameter durchgeführt werden kann. Vorliegend sind der erste und der zweite Positionsparameter PP1, PP2 als Positionswert, als Rotationswinkel oder ähnliches ausgebildet.

Erster und zweiter Positionsparameter PP1, PP2 werden vorteilhaft gleichzeitig, bzw. quasi-gleichzeitig erfasst, sodass eine Abweichung der beiden Positionsparameter PP1, PP2 spezifisch für einen Messzeitpunkt ermittelt werden kann.

In einem Schritt S04 erfolgt ein Vergleichen des ersten und des zweiten Positionsparameters PP1, PP2 mittels Recheneinheit. Es wird also geprüft, ob bzw. wie weit die erfassten Werte der Positionsparameter PP1, PP2 jeweils repräsentierend eine aktuelle Position der beweglichen Komponente BK, voneinander abweichen. Als Ergebnis kann Schritt S03 eine Abweichung oberhalb oder unterhalb eines ersten Schwellwerts S1 feststellen. Der erste Schwellwert S1 liegt bspw. abrufbar für die Recheneinheit 30 in einer dazu gehörigen Speichereinheit (nicht gezeigt) und ist bspw. spezifisch für die bewegliche Komponente BK oder eine aktuelle Betriebstemperatur oder eine Betriebsdauer.

Unterschreitet die Abweichung den ersten Schwellwert S1, wird in einem Schritt S05 mittels Recheneinheit 30 ein Bewegungssteuersignal BSS für die bewegliche Komponente BK basierend auf der dreidimensionalen Bildinformation 3DB erzeugt. Wir der erste Schwellwert S1 eingehalten, wird also erfindungsgemäß die korrekte Funktion und insbesondere korrekte Positionsanzeige für die bewegliche Komponente BK mittels Erfassungseinheit EH bestätigt, sodass die in der dreidimensionalen Bildinformation 3DB umfasste Positionsinformation weiter zur Bewegungssteuerung verwendet werden kann.

Übersteigt die Abweichung der Positionsparameterwerte den ersten Schwellwert S1, so wird in einem dazu alternativen Schritt S06 durch die Recheneinheit 30 ein Fehlersignal FS erzeugt. In Schritt S06 wird folglich eine Fehlfunktion der Erfassungseinheit EH bzw. eine Unzuverlässigkeit der gelieferten Positionsinformation in der dreidimensionalen Bildinformation 3DB angenommen. Die dreidimensionale Bildinformation 3DB wird in diesem Fall nicht zur Bewegungssteuerung verwendet, da zu unsicher.

In einem Schritt S07 wird das erzeugte Steuersignal BSS oder FS entweder durch die bewegliche Komponente BK, genauer ihre Antriebseinheit A oder durch die Recheneinheit 30 und ggf. weitere Einheiten zur Ausgabe von Signalen, bspw. der Interventionsmonitor M, ausgeführt.

Das Fehlersignal FS sieht vorliegend eine Warnsignalausgabe vor. Ein Warnsignal WS soll dabei einer Bedienperson B anzeigen, dass eine weitere Bewegungssteuerung nicht auf der dreidimensionalen Bildinformation 3DB basiert, die Gefahr einer Kollision besteht und/oder die Bewegung der beweglichen Komponente BK gestoppt wird. Alternativ oder zusätzlich kann das Warnsignal WS anzeigen, dass die bewegliche Komponente BK nur noch manuell oder nach manueller Freigabe durch die Bedienperson B bewegt werden kann. Das Warnsignal WS kann auch das Aktivieren eines üblichen Kollisionsvermeidungsverfahrens zur weiteren Bewegungssteuerung vorsehen. Dieses basiert jedoch nicht auf den in der dreidimensionalen Bildinformationen 3DB umfassten Positionsdaten zu der beweglichen Komponente BK und weiteren Objekten 0 in deren Umgebung U und ist per se beschränkt auf weitere bewegliche oder unbewegliche Komponenten der medizinischen Anlage 100, die vorab bekannt sind. Die Bewegung einer Bedienperson B kann klassisch durch eine üblichen Kollisionsvermeidungsalgorithmus nicht überwacht werden.

Um eine Bewegung der beweglichen Komponente BK auf potenzielle Kollisionen hin überwachen zu können, werden die erfindungsgemäßen Schritte S01 bis S07 selbstredend während der Bewegung der beweglichen Komponente BK ausgeführt. Das Verfahren kann bspw. aber auch bei Stillstand der beweglichen Komponente ablaufen und die Bewegung anderer Objekte überwachen und zur Kollisionsvermeidung beitragen.

Veranschaulicht durch den gestrichelten Pfeil, werden die Schritte S01 bis S07 mehrfach, also in einer Art Wiederholschleife ausgeführt. Dabei erfolgt eine besonders engmaschige, quasi kontinuierliche Kollisionsüberwachung, wenn die Schritte S01 bis S07 mit der Wiederholrate der Erfassungseinheit EH von 15 Hz (= 15fps) ausgeführt werden.

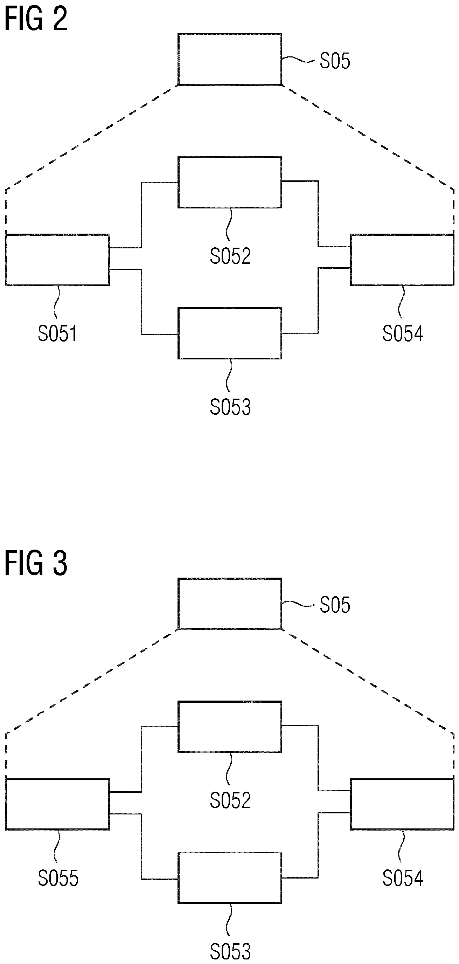

**Die** **Figuren 2, 3** **und** **4** zeigen jeweils eine Detailansicht eines Ablaufdiagramms eines Bewegungssteuerverfahrens in unterschiedlichen Ausführungsformen.

Wie eingangs bereits angeführt, umfasst die dreidimensionale Bildinformation 3DB neben der Positionsinformation für die bewegliche Komponente BK auch Positionsinformation zu weiteren dargestellten bzw. abgebildeten Objekten 0 in der direkten Umgebung U der beweglichen Komponente BK. Diese weitere Positionsinformation wird nun, da die Verlässlichkeit bzw. die Funktionalität der Erfassungseinheit EH mittels Schwellwertvergleich nachgewiesen wurde, für die Bewegungssteuerung genutzt. Dabei wird davon ausgegangen, dass die ableitbare Positionsinformation zu weiteren Objekten O genauso zutreffend ist wie die Positionsinformation bzgl. der beweglichen Komponente BK. Entsprechend wird, wie in Figur 2 dargestellt in einem Unterschritt S055 zu Schritt S05 ein Abstand, also eine Abstandsinformation bzw. ein Abstandswert zwischen der beweglichen Komponente BK und einem als Hindernis identifizierten Objekt O in der Umgebung U der beweglichen Komponente BK anhand der dreidimensionalen Bildinformation 3DB bestimmt. Dieser Schritt umfasst ebenfalls eine an sich bekannte visuelle Bildverarbeitung zur Bestimmung der Position des Objektes, sowie mit Bezug zu dem ersten Positionsparameter PP1 weiter oben bereits erläutert, um die dreidimensionale Position des Objektes zu ermitteln. Danach erfolgt eine Abstandswertberechnung basierend auf dem ersten Positionsparameter PP1 sowie der Positionsinformation des Objektes. Dieser Abstandswert wird im weiteren Verlauf des erfindungsgemäßen Verfahrens, insbesondere des Unterschritten S052, S053 berücksichtigt.

Bspw. ist Unterschritt S052 darauf gerichtet, den in Unterschritt S051 ermittelten Abstandswert mit einem ersten Abstandsschwellwert AS1 zu vergleichen. Wird dieser vorab für die jeweilige bewegliche Komponente BK definierte erste Abstandsschwellwert AS1 überschritten, der Abstand also groß genug und damit sicher ist, da sich das Objekt bspw. außerhalb einer ersten Sicherheitszone um die bewegliche Komponente BK befindet, wird in Unterschritt S054 hingegen ein Bewegungssteuersignal erzeugt, welches eine Geschwindigkeitserhöhung für die bewegliche Komponente BK vorsieht. Unterschreitet der abgeleitete Abstandswert zwischen beweglicher Komponente BK und Objekt O in Unterschritt S052 den ersten Abstandsschwellwert AS1, wird die erste Sicherheitszone der beweglichen Komponente BK also verletzt, wird in Unterschritt S054 ein Bewegungssteuersignal erzeugt, welches eine Geschwindigkeitsreduktion für die bewegliche Komponente BK vorsieht. Parallel dazu kann das Bewegungssteuersignal BSS die Ausgabe eines Warnsignals für die Bedienperson B darüber vorsehen, dass die erste Sicherheitszone bereits verletzt ist, um die Aufmerksamkeit der Bedienperson B im Hinblick auf die folgende Bewegung der beweglichen Komponente zu erhöhen.

Ein anderer, alternativer oder zusätzlicher Unterschritt S053 ist darauf gerichtet, zu prüfen, ob der in Unterschritt S051 ermittelte Abstandswert einen zweiten Abstandsschwellwert AS2 entsprechend einer zweiten Sicherheitszone um die bewegliche Komponente BK unterschreitet oder nicht. Der zweite Abstandsschwellwert AS2 ist hier kleiner als der erste Abstandsschwellwert, eine Verletzung der zweiten Sicherheitszone ist also noch kritischer als eine Verletzung der ersten Sicherheitszone. Insofern wird bei Unterschreiten des zweiten Abstandsschwellwerts in Unterschritt S053 in Unterschritt S054 ein Bewegungssteuersignal abgeleitet, dass eine Richtungsänderung, insbesondere eine Richtungsumkehrt, für die bewegliche Komponente vorsieht, um eine Kollision zu vermeiden. Erfindungsgemäß geht die bewegliche Komponente BK dem als Hindernis erkannten Objekt O aus dem Weg`. Alternativ kann das in Unterschritt S054 erzeugte Bewegungssteuersignal darauf gerichtet sein, die Bewegung der beweglichen Komponente zu stoppen.

Aus hier kann zusätzlich vorgesehen sein, bei Verletzung der zweiten Sicherheitszone ein weiteres Warnsignal an die Bedienperson B auszugeben.

In der in Figur 3 mittels weiterer Detailansicht dargestellten Ausführungsvariante des erfindungsgemäßen Bewegungssteuerverfahrens ist die bewegliche Komponente BK ein Patientenlagerungstisch PL der medizinischen Anlage 100, auf welchem ein Patient PA positioniert ist (Vgl Figur 6). In dieser Ausführung umfasst das Bestimmen des Abstands zwischen der beweglichen Komponente BK und dem als Hindernis identifizierten Objekt O, einen Abstand zwischen dem Patienten PA und dem Hindernis basierend auf der dreidimensionalen Bildinformation 3DB zu bestimmen. Mit anderen Worten umfasst ein Unterschritt S055 in dieser Ausführung die Erfassung von Positionsinformationen wie gehabt für den Patientenlagerungstisch PL und den Patienten PA, wobei der Patient PA hier der beweglichen Komponente BK zugerechnet bzw. darunter subsummiert wird. Der Patient PA wird folglich in dieser Ausführung nicht als Objekt, sondern als bewegliche Komponente BK behandelt. Anhand dieser Positionsinformationen kann nun in Bezug auf weitere mittels der dreidimensionalen Bildinformation 3DB dargestellte Objekte eine Abstandsermittlung wie oben beschrieben durchgeführt und in den weiteren Unterschritten S052, S053 und S053 ein Bewegungssteuersignal BSS ermittelt werden. Für die Ausbildung der Unterschritte S052, S053, S054 wird auf die Beschreibung der Figur 2 verwiesen.

Der Vollständigkeit sei darauf verwiesen, dass sofern eine medizinische Anlage 100 nicht selbst und vollständige die bewegliche Komponente BK bildet, jede einzelne, insbesondere jede bewegliche Komponente BK für eine andere bewegliche Komponente BK als weiteres Objekt O und damit als Hindernis wirken kann. Insbesondere ist auch der Patient PA in Bezug auf eine bewegliche Komponente BK typischerweise als Objekt O bzw. Hindernis ausgebildet. Insofern sei nochmals ohne konkreten Bezug zu einer der Figuren klargestellt, dass ein Objekt O bzw. ein Hindernis als starres oder bewegtes Hindernis ausgebildet sein kann, wobei das Objekt O bzw. das Hindernis vorzugsweise von einer nicht zu der medizinischen Anlage 100 gehörigen Einheit, aber auch durch eine weitere Komponente der medizinischen Einheit 100 gebildet werden kann. Mit dem erfindungsgemäßen Verfahren können nun insbesondere auch die unbekannten, also nicht zu der medizinischen Anlage 100 gehörigen Objekte O bei der Kollisionsvermeidung berücksichtigt werden.

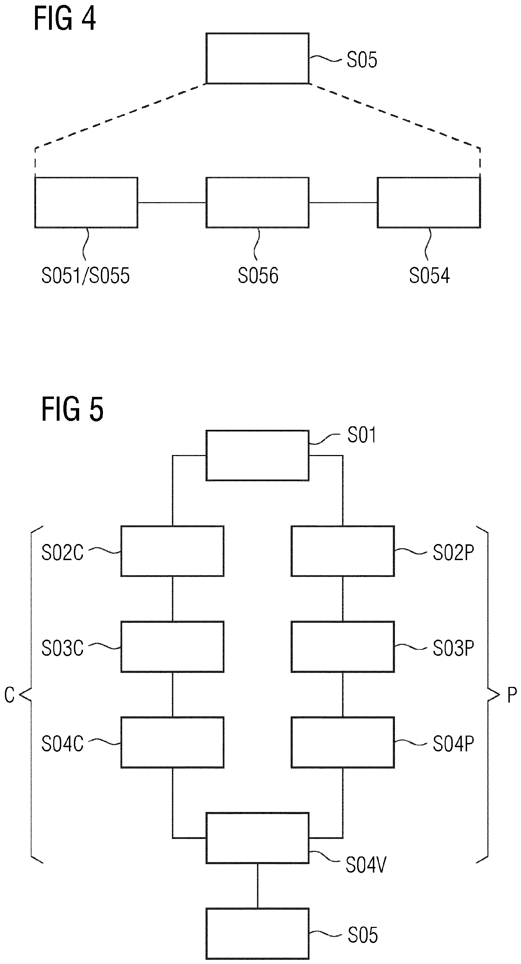

Die in Figur 4 gezeigte Detailansicht des erfindungsgemäßen Steuerverfahrens adressiert eine weitere Ausführungsform. Hierbei erfolgt eine Abstandswertberechnung in Unterschritt S051 oder S055 wie in Figur 2 oder 3 beschrieben. Die Unterschritte S053 und S054 werden hier jedoch ersetzt durch Unterschritt S056, der auf das Aktivieren eines klassischen Kollisionsvermeidungsverfahrens gerichtet ist und darauf, den berechneten Abstandswert bzw. die aus der dreidimensionalen Bildinformation ermittelte Positionsinformation zu einem Objekt 0 an diesen zu übertragen. Der Kollisionsvermeidungsalgorithmus ist bspw. abrufbar bzw. aktivierbar in der Speichereinheit der Recheneinheit 30 hinterlegt. Diese wird mit der Abstandsinformation bzw. der Positionsinformation aktualisiert, insbesondere im Hinblick auf ein ihm bislang unbekanntes Objekt O, wodurch die in dem Verfahren umfasste Pfadplanung und Kollisionsvermeidungsvorschriften erweitert und verbessert wird. Unterschritt S054 zur Erzeugung eines Bewegungssteuersignals kann dann wieder wie in den Figuren 2 oder 3 beschrieben, ausgeführt sein.

**Figur 5** zeigt eine weitere Detailansicht eines Ablaufdiagramms eines Bewegungssteuerverfahrens in einer besonders bevorzugten Ausführungsform. In dieser Ausführung werden die Schritte S02, S03 und S04, also die Überprüfung der Funktionalität der Erfassungseinheit EH, redundant und unabhängig voneinander ausgeführt, um die Sicherheit und die Verlässlichkeit des Bewegungssteuerverfahrens weiter zu erhöhen. Die Recheneinheit 30 umfasst in entsprechenden Ausführungen zwei unabhängige Rechenmodule, eine Steuermodul C (= Control) zur Ausbildung eines Steuerpfads und einen Schutzmodul P (= Protect) zur Ausbildung eines Schutzpfads. In beiden Rechenmodulen werden die S02 bis S04 parallel nebeneinander ausgeführt. Im Steuerteil C wird folglich die Programmroutine zur Ausführung der Rechenschritte S02C, S03C, S04C durchlaufen. Im Schutzteil P wird die Programmroutine zur Ausführung der Rechenschritte S02P, S03P, S04P durchlaufen, wobei die Einzeloperationen jeweils identisch ausgebildet sein können und den Schritten S02 bis S04, wir mit Bezug zu Figur 1 beschrieben, ausgebildet sein können. In einem Vergleichsschritt S04V erfolgt ein Vergleich der Vergleichsergebnisse der unabhängigen Vergleichsschritte S04C, S04P. Nur für den Fall einer Übereinstimmung und einer Bestätigung des Steuerpfads durch den Schutzpfad, erfolgt im weiteren Schritt S05, wie oben beschrieben, die Erzeugung eines Bewegungssteuersignals BSS unter Verwendung und weiterer Auswertung der dreidimensionalen Bildinformation BSS. Kann in Schritt S04V keine Übereinstimmung der Vergleichsdaten festgestellt werden, geht wird bspw. das Verfahren gemäß Schritt S06 fortgesetzt.

**Figur 6** zeigt eine Ansicht einer medizinischen Anlage 100 in Form einer Bildgebungs- bzw. Interventionsanlage, kurz einer Angiographie-Anlage gemäß einer Ausführungsform der vorliegenden Erfindung. Die Angiographie-Anlage 100 ist repräsentativ für eine Vielzahl von verschiedenen Anlagen, und nicht einschränkend zu verstehen. Die Angiographie-Anlage wurde aufgrund ihrer vergleichsweise vielen Bewegungsfreiheitsgrade zur Veranschaulichung gewählt. Die medizinische Anlage 100 kommt bevorzugt für Angiographie-Untersuchungen oder Angiographie-Eingriffe zum Einsatz. Andere Ausbildungen der medizinischen Anlage 100 sind aber ebenfalls denkbar und im Sinne der Erfindung.

Die medizinische Anlage 100 umfasst eine Patientenliege bzw. einen Patientenlagerungstisch PL zur Aufnahme bzw. Positionierung eines Patienten PA. Dieser kann, muss aber nicht, von einem Abdecktuch bedeckt sein. Die medizinische Anlage 100 umfasst weiter eine Röntgenstrahlenquelle RQ und einen Röntgenstrahlendetektor RD. Röntgenstrahlenquelle RQ und Röntgenstrahlendetektor RD sind an sich gegenüberliegenden Enden eines C-Arms CA der Anlage 100 angeordnet. Der C-Arm CA selbst kann um mehrere Rotationsachsen um den Patienten PA herum rotieren, Daneben können Röntgenstrahlenquellen RQ und Röntgenstrahlendetektor RD entlang der Orbitalbahn des C-Arms CA verstellt werden, um aus verschiedenen Perspektiven Röntgenbilddaten eines abzubildenden Körperbereich des Patienten PA zu erfassen. Die Patientenliege PL weist eine Auflagefläche AF auf, auf der der Patient PA platziert werden kann. Auch die Patientenliege PL ist verstellbar und damit im Raum bewegbar. Bspw. kann die Auflagefläche AF samt Patient PA entlang ihrer Längsachse verschoben werden. Daneben kann über das Vertikalmodul die Auflagefläche AF samt Patient PA in der Höhe verstellt werden.

Die medizinische Anlage 100 umfasst weiter einen deckengehängten, positionierbaren Interventionsmonitor M in dichter räumlicher Nähe zu dem C-Arm CA.

In direkter räumlicher Nähe zu dem Patienten PA bzw. dem C-Arm befindet sich auch eine Bedienperson B, die bspw. einen medizinischen Eingriff am Patienten unter Röntgenbildgebung mittels medizinischen Anlage 100 durchführt. Dazu benötigt die Bedienperson B bspw. medizinische Instrumente, die bspw. auf einem mobilen, ggf. sogar autonom fahrbaren Instrumentenwagen IW in die Untersuchungsumgebung U gebracht werden.

Die medizinische Anlage 100 umfasst folglich wenigsten eine, hier drei bewegliche Komponenten BK im Sinne der Erfindung, den C-Arm CA, die Patientenliege PL sowie den deckengehängten Interventionsmonitor M.

In der Umgebung U bzw. in direkter räumlicher Nähe zu den jeweiligen beweglichen Komponenten BK sind bewegliche oder unbewegliche Objekte O angeordnet, bspw. die sich bewegende Bedienperson B und der fahrbare Instrumentenwagen IW, die eine Bewegung einer beweglichen Komponente BK stören und zu einer Kollision führen können. Neben dem rein der Veranschaulichung dienenden Instrumentenwagen IW können auch andere, nicht dargestellte starre oder bewegliche Objekte in der Umgebung U angeordnet sein, bspw. Beatmungs- oder EKG-Geräte, Strahlenschutzvorrichtungen oder dergleichen.

Für jede der beweglichen Komponenten BW kann nun das oben beschriebene, erfindungsgemäße Verfahren durchgeführt werden.

Zu diesem Zweck umfasst die medizinische Anlage 100 eine Erfassungseinheit EH, die hier zwei 3D-Kameras K1, K2 umfasst. Diese sind beide an der Raumdecke, im Wesentlichen oberhalb der Patientenliege PL mit nach unten gerichtetem Detektionsfeld positioniert. Die Detektionsfelder der beiden 3D-Kameras K1, K2 ergänzen sich vorteilhaft, sodass mittels beider 3D-Kameras K1, K2 ein größeres Detektionsfeld abgedeckt wird.

Dies ist insbesondere vorteilhaft für medizinische Anlagen mit einer Vielzahl von beweglichen Komponenten BK und/oder einem vergleichsweise großen Bewegungsradius. In anderen Ausführungen kann die Erfassungseinheit EH auch nur eine 3D-Kamera aufweisen. Die Erfassungseinheit EH ist ausgebildet, eine dreidimensionale Bildinformation 3DB von wenigstens einer beweglichen Komponente BK und ihrer Umgebung U zu erfassen. Mit anderen Worten liefert die Erfassungseinheit neben einem zweidimensionalen Bild auch Tiefeninformation zu jedem Pixel des 2D-Bilds. Konkret liefert eine 3D-Kamera insbesondere mit einer definierten Widerholrate, bspw. 15fps, also 15 Mal pro Sekunde, ein RGB-Bild sowie ein dazugehöriges Tiefenbild. In der Umgebung U können nun weitere Objekte O wie oben beschrieben vorhanden und in der dreidimensionalen Bildinformation 3DB mit umfasst sein. Derart ermöglicht die Erfassungseinheit eine kontinuierliche Kollisionsüberwachung.

Die medizinische Anlage 100 umfasst ferner eine Recheneinheit 30, die ausgebildet ist, basierend auf der erfassten dreidimensionalen Bildinformation Steuersignale für die medizinische Anlage 100 zu erzeugen. Steuersignale im Sinne der Erfindung können Signale zur Bewegungssteuerung, aber auch Fehlersignale umfassen, die weiter oben jeweils schon näher beschrieben werden. Die Steuersignale dienen jeweils der Bewegung der wenigstens einen beweglichen Komponente BK bzw. einer Kollisionsvermeidung bzw. einer Kollisionsüberwachung. Das in der Recheneinheit 30 ausgeführte Verfahren ist vorteilhaft vollautomatisch und sicher ausgeführt. Die Recheneinheit 30 liefert basierend auf der dreidimensionalen Bildinformation 3DB für die Antriebseinheit A wenigstens einer der beweglichen Komponenten BK, um diese sicher und kollisionsfrei zu bewegen. Die Recheneinheit ist folglich zum Ermitteln eines ersten Positionsparameters PP1 zu einer der beweglichen Komponenten BK basierend auf der dreidimensionalen Bildinformation 3DB, zum Erfassen eines zweiten Positionsparameters PP2 zu der beweglichen Komponente BK, zum Vergleichen des ersten und des zweiten Positionsparameters PP1, PP2 und zum Erzeugen eines Bewegungssteuersignals BSS für die bewegliche Komponente BK basierend auf der dreidimensionalen Bildinformation 3DB ausgebildet, sofern die Abweichung zwischen erstem und zweitem Positionsparameter PP1, PP2 einen vorab definierten ersten Schwellwert S1 unterschreitet. Die Recheneinheit 30 ist ferner ausgebildet, ein Fehlersignals FS zu erzeugen, sofern die Abweichung zwischen erstem und zweitem Positionsparameter PP1, PP2 den ersten Schwellwert S1 überschreitet. Die Recheneinheit 30 ist ferner ausgebildet, das Fehlersignal FS auszuführen. Ein Bewegungssteuersignal BSS wird insbesondere von der zu der beweglichen Komponente BK gehörigen Antriebseinheit A ausgeführt. Zur Ausführung des Fehlersignals FS kann die Recheneinheit mit weiteren Einheiten, bspw. dem Monitor M, zur Ausgabe eines Warnhinweises an die Bedienperson B zusammenwirken.

Zum Erfassen des zweiten Positionsparameters PP2 umfasst die medizinische Anlage eine hier nur exemplarisch veranschaulichte Sensoreinheit SE. Die Sensoreinheit SE der medizinischen Anlage 100 umfasst weiter wenigstens einen Sensor zum Erfassen einer Positionsinformation zu einer der beweglichen Komponenten BK. Die Sensoreinheit weist bevorzugt einen Sensor in einem Antriebsstrang einer beweglichen Komponente auf. Der Sensor kann als Positionssensor und/oder als Winkelsensor oder dergleichen ausgebildet sein. Ein derartiger Sensor ist standardmäßig in medizinischen Anlage 100 bereits zur routinemäßigen Positionsüberwachung verbaut.

Die Recheneinheit 30 steht in der gezeigten Ausführung mit der wenigstens einen Antriebseinheit A, der Erfassungseinheit EH und der Sensoreinheit SE über wenigstens eine Datenschnittstelle (nicht gezeigt) in uni- oder bidirektionaler Datenkommunikation. Die Datenschnittstelle kann kabelgebunden oder kabellos, jeweils auf an sich bekannte Weise, ausgebildet sein. Die Recheneinheit 30 ist ausgebildet, Sensorsignale der Sensoreinheit SE sowie der Erfassungseinheit EH zu empfangen und zu verarbeiten. Sie ist weiter ausgebildet, Steuerbefehle/Steuersignale bspw. entsprechend einem Bewegungssteuersignal BSS oder einem Warnsignal für eine Antriebseinheit A oder den Monitor M zu erzeugen und über die Datenschnittstelle zu übertragen. Insbesondere ist die Recheneinheit 30 ausgebildet, Steuerbefehle in Abhängigkeit einer Auswertung der erfassten dreidimensionalen Bildinformation 3DB zu bestimmen und auszusenden. Die Recheneinheit 30 ist insbesondere ausgebildet, anhand oder aus der dreidimensionalen Bildinformation 3DB Abstandsinformationen bezüglich einer der beweglichen Komponenten BK und wenigstens einem weiteren in der Umgebung U befindlichen Objekt O zu ermitteln und basierend darauf ein Bewegungssteuersignal BSS abzuleiten.

Recheneinheit 30, Erfassungseinheit EH und Sensoreinheit SE sind hier als Teil einer Anlagensteuervorrichtung der medizinischen Anlage 100 ausgebildet. Die Recheneinheit 30 kann in Form von Hard- oder in Form von Software ausgebildet sein. Beispielsweise ist die Recheneinheit 30 als ein sogenanntes FPGA (Akronym für das englischsprachige "Field Programmable Gate Array") ausgebildet oder umfasst eine arithmetische Logikeinheit.

In der hier gezeigten Ausführungsform ist in der Speichereinheit der Recheneinheit 30 wenigstens ein Computerprogrammprodukt gespeichert, welches Verfahrensschritte des erfindungsgemäßen Verfahrens durchführt, wenn das Computerprogrammprodukt auf der Recheneinheit 30 ausgeführt wird. Das Computerprogrammprodukt zur Ausführung von Verfahrensschritten des erfindungsgemäßen Verfahrens umfasst Programmcode. Weiterhin kann das Computerprogramm als ausführbare Datei ausgebildet sein und/oder auf einem anderen Rechensystem als der Recheneinheit 30 gespeichert sein. Beispielsweise kann die medizinische Anlage 100 so ausgelegt sein, dass die Recheneinheit 30 das Computerprogrammprodukt zum Ausführen des erfindungsgemäßen Verfahrens über ein Intranet oder über das Internet in seinen internen Arbeitsspeicher lädt.

Im Folgenden wird die Erfindung nochmals kurz zusammengefasst:
Die vorliegende Erfindung ermöglicht es, einen aktuellen Abstand der beweglichen Komponente zu einem Hindernis erstfehlersicher als analogen Abstandswert vor einem Kollisionsereignis zu berechnen und vorher eine Sicherheitsreaktion (bspw. Reduzierung der Geschwindigkeit, Alarmton, etc.) einzuleiten.

Sowohl 3D-Kameras als auch Schutz-Pfad-Rechenmodule sind zu niedrigen Kosten verfügbar oder bereits in medizinischen Anlagen verbaut. Die Erfindung lässt sich folglich weitgehend kostenneutral realisieren.

Das Vorsehen einer 3D-Kamera in medizinischen Anlagen, in denen bislang keine verbaut wurde, wirkt sich weiter positiv auf den sonstigen medizinischen Workflow aus. Denn jetzt kann "kostenneutral" der Patient sehr exakt vermessen werden. Dazu muss der Patient an sich vermessen werden, bevor seine Kontur bspw. durch ein Abdecktuch verdeckt/verändert wird, was problemlos möglich ist, da die 3D-Kamera durchgehend misst. Ein höherer Automatisierungsgrad ist die Folge. Bewegungen beweglicher Komponenten können nun besser auf das Ziel der Bewegung (bspw. Herz) durchgeführt werden. Neben der Kollisionsüberwachung erlaubt die Erfindung also auch eine Workflowautomatisierung werden.

Der permanent wiederholte Funktionstest für die 3D-Kamera erlaubt auch eine Überprüfung der Patientenposition bzw. seiner Lage. Eine initial, also zu Beginn eines medizinischen Workflows erfasste Patientenposition kann leicht und jederzeit mit einer aktuellen Patientenposition verglichen werden. Bei Positionsabweichung kann der Bediener informiert und die Patientenposition korrigiert werden.

Durch die Anwendung des erfindungsgemäßen Verfahrens können viele der mechanischen Sensoren entfallen, die bislang für eine Kollisionsüberwachung vorgesehen werden musste, die Teilekomplexität sinkt und die medizinische Anlage wird optisch ansprechender. Erfindungsgemäß können alle Kanten und Flächen der medizinischen Anlage/der beweglichen Komponente geschützt werden, die erfindungsgemäße Kollisionsüberwachung beschränkt sich nicht auf ausgewählte oder besonders gefährdete Kanten und Flächen. Erfindungsgemäß wird sicher ein aktueller Abstand ermittelt, sodass eine Kollision vorhergesagt werden kann. Dadurch kann außerhalb einer Kollisionsgefahr eine Bewegung der beweglichen Komponente mit höheren Geschwindigkeiten als bislang erfolgen. Innerhalb von Sicherheitszonen kann eine Bewegung zwar mit geringerer Geschwindigkeit, aber dennoch fortgesetzt werden (= Proximity-Funktion). Kommt es, bspw. aufgrund einer Ungenauigkeit der 3D-Kamera-Messung dennoch zu einer Kollision, erfolgt diese lediglich mit sehr geringer Geschwindigkeit, so dass trotz eines Kollisionsereignisses die Gefährdung stark reduziert ist.

Obwohl die Erfindung im Detail durch das bevorzugte Ausführungsbeispiel näher illustriert und beschrieben wurde, ist die Erfindung nicht durch dieses Ausführungsbeispiel eingeschränkt. Andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Bewegungssteuerverfahren für eine bewegliche Komponente (BK, CA, M, PL) einer medizinischen Anlage (100), umfassend die Schritte
i) Erfassen (S01) einer dreidimensionalen Bildinformation (3DB) von der beweglichen Komponente (BK) und ihrer Umgebung (U) mittels einer Erfassungseinheit (EH)
ii) Ermitteln (S02) eines ersten Positionsparameters, (PP1) zu der beweglichen Komponente (BK) basierend auf der dreidimensionalen Bildinformation (3DB) mittels einer Recheneinheit (30),
iii) Erfassen (S03) eines zweiten Positionsparameters (PP2) zu der beweglichen Komponente (BK) mittels Recheneinheit (30),
iv) Vergleichen (S04) des ersten und des zweiten Positionsparameters (PP1, PP2) mittels Recheneinheit (30),
v) Erzeugen (S05) eines Bewegungssteuersignals (BSS) für die bewegliche Komponente (BK) basierend auf der dreidimensionalen Bildinformation (3DB), sofern die Abweichung zwischen erstem und zweitem Positionsparameter (PP1, PP2) einen vorab definierten ersten Schwellwert (S1) unterschreitet mittels Recheneinheit (30),
vi) Erzeugen (S06) eines Fehlersignals (FS), sofern die Abweichung zwischen erstem und zweitem Positionsparameter (PP1, PP2) den ersten Schwellwert (S1) überschreitet mittels Recheneinheit (30), und
vii) Ausführen (S07) des Bewegungssteuersignals (BSS) durch die bewegliche Komponente (BK) oder des Fehlersignals (FS) durch die Recheneinheit (30).

2. Bewegungssteuerverfahren nach Anspruch 1, wobei das Erzeugen (S05; S051; S055) des Bewegungssteuersignals (BSS) umfasst, einen Abstand zwischen der beweglichen Komponente (BK) und einem Hindernis in der Umgebung der beweglichen Komponente (BK) anhand der dreidimensionalen Bildinformation (3DB) zu bestimmen.

3. Bewegungssteuerverfahren nach Anspruch 2, wobei das Bewegungssteuersignal (BSS) den Abstand zwischen der beweglichen Komponente (BK) und dem Hindernis berücksichtigt.

4. Bewegungssteuerverfahren nach Anspruch 2 oder 3, wobei die bewegliche Komponente (BK) ein Patientenlagerungstisch (PL) ist, auf welchem ein Patient (PA) positioniert ist, wobei das Bestimmen (S055) des Abstands zwischen der beweglichen Komponente (BK) und dem Hindernis umfasst, einen Abstand zwischen dem Patienten (PA) und dem Hindernis basierend auf der dreidimensionalen Bildinformation (3DB) zu bestimmen.

5. Bewegungssteuerverfahren nach einem der vorherigen Ansprüche, wobei das Bewegungssteuersignal (BSS) eine Geschwindigkeitserhöhung für die bewegliche Komponente (BK) vorsieht, wenn der Abstand zwischen beweglicher Komponente (BK) und Hindernis einen ersten Abstandsschwellwert (AS1) überschreitet und eine Geschwindigkeitsreduktion für die bewegliche Komponente (BK) vorsieht, wenn der Abstand zwischen beweglicher Komponente (BK) und Hindernis den ersten Abstandsschwellwert (AS1) unterschreitet.

6. Bewegungssteuerverfahren nach einem der vorherigen Ansprüche, wobei das Bewegungssteuersignal (BSS) eine Richtungsänderung für die bewegliche Komponente (BK) vorsieht, wenn der Abstand zwischen beweglicher Komponente (BK) und Hindernis einen zweiten Abstandsschwellwert (AS2) unterschreitet.

7. Bewegungssteuerverfahren nach einem der vorherigen Ansprüche, wobei das Fehlersignal (FS) eine Warnsignalausgabe und/oder das Aktivieren (S06) eines Kollisionsvermeidungsverfahrens vorsieht.

8. Bewegungssteuerverfahren nach einem der vorherigen Ansprüche, wobei das Hindernis als starres oder bewegtes Hindernis ausgebildet ist.

9. Bewegungssteuerverfahren nach einem der vorherigen Ansprüche, wobei das Hindernis von einer nicht zu der medizinischen Anlage (100) gehörigen Einheit gebildet wird.

10. Bewegungssteuerverfahren nach einem der vorherigen Ansprüche, wobei der erste und/oder der zweite Positionsparameter (PP1, PP2) der beweglichen Komponente (BK) als Positionswert oder als Rotationswinkel ausgebildet sind.

11. Bewegungssteuerverfahren nach einem der vorherigen Ansprüche, wobei die Schritte i) bis vii) während einer Bewegung der beweglichen Komponente (BK) ausgeführt werden.

12. Bewegungssteuerverfahren nach einem der vorherigen Ansprüche, wobei die Schritte i) bis vii) mit 15 Hz wiederholt werden.

13. Bewegungssteuerverfahren nach einem der vorherigen Ansprüche, wobei die Schritte ii), iii) und iv) redundant und unabhängig voneinander ausgeführt werden.

14. Bewegungssteuerverfahren nach einem der vorherigen Ansprüche 2 bis 10, wobei Schritt v) umfasst, das Bewegungssteuersignal (BSS) unter Anwendung (S056) eines Kollisionsvermeidungsverfahrens zu erzeugen, welches den ermittelten Abstand zwischen beweglicher Komponente (BK) und Hindernis berücksichtigt.

15. Medizinische Anlage (10), ausgebildet zum Ausführen eines Bewegungssteuerverfahrens nach einem der Ansprüche 1 bis 14, umfassend
- eine bewegliche Komponente (BK),
- eine Erfassungseinheit (EH) ausgebildet zum Erfassen einer dreidimensionalen Bildinformation (3DB) von der beweglichen Komponente (BK) und ihrer Umgebung (U),
- eine Recheneinheit (30) ausgebildet zum Ermitteln eines ersten Positionsparameters (PP1) zu der beweglichen Komponente (BK) basierend auf der dreidimensionalen Bildinformation (3DB),
- Erfassen eines zweiten Positionsparameters (PP2) zu der beweglichen Komponente (BK),
- Vergleichen des ersten und des zweiten Positionsparameters (PP1, PP2),
- Erzeugen eines Bewegungssteuersignals (BSS) für die bewegliche Komponente (BK) basierend auf der dreidimensionalen Bildinformation (3DB), sofern die Abweichung zwischen erstem und zweitem Positionsparameter (PP1, PP2) einen vorab definierten ersten Schwellwert (S1) unterschreitet,
- Erzeugen eines Fehlersignals (FS), sofern die Abweichung zwischen erstem und zweitem Positionsparameter (PP1, PP2) den ersten Schwellwert () überschreitet, und zum Ausführen des Fehlersignals (FS).

16. Medizinische Anlage (100) nach Anspruch 15, ferner umfassend eine Sensoreinheit (SE) umfassend wenigstens einen Sensor zum Erfassen des zweiten Positionsparameters (PP2) der beweglichen Komponente (BK).

17. Medizinische Anlage (100) nach Anspruch 15 oder 16, wobei die Erfassungseinheit (EH) eine 3D-Kamera (K1, K2) umfasst.

18. Computerprogrammprodukt, welches direkt in eine Speichereinheit einer Recheneinheit (30) ladbar ist, mit Programmabschnitten, um Schritte des Verfahrens nach einem der Ansprüche 1 bis 14 auszuführen, wenn das Computerprogramm in der Recheneinheit (30) ausgeführt wird.

19. Computerlesbares Medium, auf welchem von einer Recheneinheit (30) einlesbare und ausführbare Programmabschnitte gespeichert sind, um Schritte des Verfahrens nach einem der Ansprüche 1 bis 14 auszuführen, wenn die Programmabschnitte von der Recheneinheit (30) ausgeführt werden.
